(19) Europäisches Patentamt
European Patent Office
Office européen des brevets

(11) **EP 3 887 585 B1**

(12) **EUROPEAN PATENT SPECIFICATION**

(45) Date of publication and mention
of the grant of the patent:
**24.08.2022 Bulletin 2022/34**

(21) Application number: **19824073.1**

(22) Date of filing: **26.11.2019**

(51) International Patent Classification (IPC):
*D04H 3/16* (2006.01)     *D04H 3/147* (2012.01)
*D04H 3/14* (2012.01)     *A61F 13/511* (2006.01)
*A61F 13/472* (2006.01)

(52) Cooperative Patent Classification (CPC):
**D04H 3/16; A61F 13/472; A61F 13/51121;
D04H 3/14; D04H 3/147;** A61F 2013/51028

(86) International application number:
**PCT/US2019/063142**

(87) International publication number:
**WO 2020/112705 (04.06.2020 Gazette 2020/23)**

(54) **THROUGH-FLUID BONDED CONTINUOUS FIBER NONWOVEN WEBS**

DURCHSTRÖMUNGSFLÜSIGKEITSGEBUNDENE ENDLOSFASERVLIESBAHNEN

BANDES NON-TISSÉES DE FIBRES CONTINUES LIÉES PAR FLUIDE TRAVERSANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.11.2018 US 201862773228 P**

(43) Date of publication of application:
**06.10.2021 Bulletin 2021/40**

(73) Proprietor: **The Procter & Gamble Company**
**Cincinnati, OH 45202 (US)**

(72) Inventors:
• **BRENNAN, Jonathan P.**
**Cincinnati, Ohio 45202 (US)**
• **AUER, Jeffrey A.**
**Cincinnati, Ohio 45202 (US)**
• **MONEBRAKE, David Wesley**
**Cincinnati, Ohio 45202 (US)**
• **DEBEER, Antonius Lambertus**
**Loveland, Ohio 45140 (US)**

(74) Representative: **P&G Patent Germany**
**Procter & Gamble Service GmbH**
**Sulzbacher Straße 40**
**65824 Schwalbach am Taunus (DE)**

(56) References cited:
**WO-A1-01/12888     WO-A1-2005/001188**

**Description**

FIELD

The present disclosure is generally directed to through-fluid bonded continuous fiber nonwoven webs, and is more particularly directed to absorbent articles comprising through-fluid bonded continuous fiber nonwoven webs.

BACKGROUND

Absorbent articles, such as diapers, pants, adult incontinence products, sanitary napkins, and liners use nonwoven webs as various components. Some example components using nonwoven webs are topsheets, outer cover nonwoven materials, ears, side panels, leg cuffs, and landing zones, for example. Consumers desire nonwoven webs that are soft and lofty, but that do not cause "fuzz" on a wearer or caregiver. Current nonwoven webs struggle to provide soft and lofty and non-fuzzing webs that have adequate strength. Nonwoven webs typically either comprise carded fibers or continuous fiber. Carded fiber nonwoven webs provide better softness than continuous fiber nonwoven webs, but are much more expensive to produce. Continuous fiber nonwoven webs are not as soft, but are cheaper to produce. The continuous fiber nonwoven webs may be manufactured by a continuous fiber nonwoven manufacturing operation. The continuous fibers may comprise multi-constituent fibers such as bicomponent fibers or tricomponent fibers, for example.

In the manufacturing operation, continuous fiber strands of molten polymer may be drawn or pushed downwardly from a spinneret by a fluid, such as air, toward a moving porous member, such as a moving porous belt. During the drawing or pushing, the continuous fiber strands may be quenched and stretched. Once the continuous fibers are deposited on the moving porous member, they may be formed into an intermediate continuous fiber nonwoven web and may be conveyed downstream facilitated by various methods of control for final bond to produce a finished continuous fiber nonwoven web. An "intermediate continuous fiber nonwoven web" as used herein means a web that has not yet been finally bonded. After the continuous fiber strands are quenched and stretched the continuous fiber strands may bend, curl, and/or twist once tension on a continuous fiber strand applied either by the stretching, air or moving porous member vacuum, has been removed. This is referred to as "self-crimping." The amount of bend, curl, and/or twist may be varied based on composition as well as quenching and stretching process conditions. Under the right process conditions, continuous fiber strands with a high degree of crimping may be used to form an unbonded and lofty continuous fiber nonwoven web on the moving porous member. However, if the continuous fiber strands are allowed to self-crimp too much before final bonding, the intermediate continuous fiber nonwoven web may fail to have sufficient integrity to be conveyed reliably on the moving porous member or become non-uniform in formation with a significant reduction in strength and softness or other properties in addition to having an undesirable non-uniform appearance. WO 2005/001188 discloses strong, high loft, low density nonwoven webs for diapers, in the form of continuous bicomponent fiber nonwovens which have been subjected to a through-fluid bonding step.

Current approaches to limit and control the loft generated by the self-crimping fibers typically includes a heated compaction process step or pre-bonding via a hot air knife prior to through-fluid bonding. However, in these approaches the lofting and softness potential of the self-crimping fibers may be reduced. In order to achieve better loft, strength, softness, and entanglement of the continuous fibers, conventional methods of producing continuous fiber nonwoven webs should be improved to achieve nonwoven webs with better loft and softness, without fuzzing or giving up strength.

SUMMARY

The present invention relates to an absorbent article as defined in claims 1-14. The present disclosure solves the problems addressed above and provides continuous fiber nonwoven webs and absorbent articles comprising the same, wherein the continuous fiber nonwoven webs have improved loft and softness without fuzzing or giving up strength. These continuous fiber nonwoven webs achieve the softness of carded nonwoven webs, but are much cheaper to produce. The present disclosure provides methods of producing these continuous fiber nonwoven webs that have improved loft, strength, and softness, via improved continuous fiber entanglement and through-fluid bonding. The present disclosure teaches that intermittently applying vacuum (e.g., turn on/off, apply/reduce) to portions of a moving porous member where the continuous fibers are laid down allows the continuous fibers to reorient relative to each other (i.e., better entangle) as the vacuum is turned off or reduced. Continuous fiber entanglement may increase the z-direction resilience of the nonwoven web for improved loft and softness after through-fluid bonding. Vacuum may be turned on/off as many times in zones along the moving porous member as necessary to achieve desirable fiber entanglement. This may comprise turning the vacuum on/off (or apply/reduce ) as many as 15 times, as many as 10 times, as many as 7 times, as many as 5 times, as many as 4 times, as many as 3 times, as many as 2 times, or just 1 time, for example. Instead of turning the vacuum off, the vacuum may instead merely be intermittently reduced. Stated another way, the vacuum force applied to the moving porous member and the intermediate continuous fiber nonwoven web may be a first force in certain zones and a second force in certain other zones, wherein the first force is greater than the second force. Instead of turning the vacuum on/off or varying the vacuum force, a vacuum diverter may be positioned to block vacuum from contacting the intermediate continuous fiber nonwoven web in certain zones of the moving porous member. The vacuum diverter may define zones of apertures where a fluid may apply a vacuum force to the web and other zones of non-apertures where the fluid cannot apply a vacuum force to the web. The zones of apertures may be varied in a machine direction or in a cross-machine direction. The reorienting of the continuous fibers may be aided by the fibers being crimped fibers. Crimping may occur more in zones where the vacuum is reduced, blocked, or off. Once the continuous fibers are reoriented, they may be through-fluid bonded on at least one side to produce a strong web with less fuzz, but that is still quite lofty and soft. Prior to the through-fluid bonding, the intermediate continuous fiber nonwoven web may also be intermittently heated and/or cooled with air or other mechanisms to again promote further reorienting of the continuous fibers within the web. This may improve continuous fiber contact points within the web and/or increase the entanglement of the continuous fibers in the web before final through-fluid bonding. This may comprise heating and cooling the nonwoven web above and below the glass transition temperature of at least one of the continuous fiber's constituent polymers. This again may lead to improved loft and softness and improved through-fluid bonding leading to better structural integrity in the web.

During the through-fluid bonding process, while the temperature of the continuous fibers is increasing, but prior to fiber-to-fiber bonding, the continuous fibers may crimp more and/or reorient further thereby increasing the loft of the unbonded nonwoven web. This may also be accomplished via a separate pre-heating step.

While through-fluid bonding is desirable, other means of thermal bonding such as thermal point bonding may also provide improved loft and softness. Combinations of through-fluid bonding and thermal point bonding may also be desirable.

The continuous fiber nonwoven webs of the present disclosure may have a Martindale Average Abrasion Resistance Grade in the range of about 1.0 to about 3.5, about 1.0 to about 3.0, about 1.0 to about 2.9, about 1.0 to about 2.8, about 1.0 to about 2.7, about 1.0 to about 2.6, about 1.0 to about 2.5, about 1.0 to about 2.4, about 1.0 to about 2.3, about 1.0 to about 2.2, about 1.0 to about 2.1 about 1.0 to about 2, or about 1.0 to about 1.5, according to the Martindale Abrasion Resistance Grade Test. The continuous fiber nonwoven webs of the present disclosure may have a DMA Compression Resiliency in the range of about 25% to about 90%, about 25% to about 70%, about 30% to about 70%, about 25% to about 50%, or about 30% to about 50%, according to the DMA Compression Resiliency Test. The continuous fiber nonwoven webs of the present disclosure may have a Thickness in the range of about 0.5mm to about 4mm, about 0.5mm to about 3mm, about 0.5mm to about 2.5mm, or about 0.5mm to about 2mm, according to the Thickness Test. The continuous fiber nonwoven webs of the present disclosure may have a Basis Weight in the range of about 10 gsm to about 100 gsm, about 14 gsm to about 80 gsm, about 15 gsm to about 40 gsm, about 15 gsm to about 30 gsm, about 20 gsm to about 30 gsm, or about 20 gsm to about 25 gsm, according to the Basis Weight Test. The continuous fiber nonwoven webs of the present disclosure may have a Specific Nonwoven Volume in the range of about 25 $cm^3$/g to about 100 $cm^3$/g, about 30 $cm^3$/g to 100 $cm^3$/g, about 25 $cm^3$/g to about 80 $cm^3$/g, about 30 $cm^3$/g to about 80 $cm^3$/g, about 40 $cm^3$/g to about 80 $cm^3$/g, or about 25 $cm^3$/g to about 55 $cm^3$/g, about 45 $cm^3$/g to about 55 $cm^3$/g. The continuous fiber nonwoven webs may be used in an absorbent article comprising a topsheet, a backsheet, and an absorbent core positioned at least partially intermediate the topsheet and the backsheet.

BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:

Fig. 1 is a plan view of an example absorbent article in the form of a taped diaper, garment-facing surface facing the viewer, in a flat laid-out state;
Fig. 2 is a plan view of the example absorbent article of Fig. 1, wearer-facing surface facing the viewer, in a flat laid-out state;
Fig. 3 is a front perspective view of the absorbent article of Figs. 1 and 2 in a fastened position;
Fig. 4 is a front perspective view of an absorbent article in the form of a pant;
Fig. 5 is a rear perspective view of the absorbent article of Fig. 4;
Fig. 6 is a plan view of the absorbent article of Fig. 4, laid flat, with a garment-facing surface facing the viewer;
Fig. 7 is a cross-sectional view of the absorbent article taken about line 7-7 of Fig. 6;
Fig. 8 is a cross-sectional view of the absorbent article taken about line 8-8 of Fig. 6;
Fig. 9 is a plan view of an example absorbent core or an absorbent article;
Fig. 10 is a cross-sectional view, taken about line 10-10, of the absorbent core of Fig. 9;
Fig. 11 is a cross-sectional view, taken about line 11-11, of the absorbent core of Fig. 10;

Fig. 12 is a plan view of an example absorbent article of the present disclosure that is a sanitary napkin;

Fig. 12A is a perspective view of a wipe of the present disclosure;

Fig. 13 is a diagrammatic view of an apparatus for performing a process for producing a through-fluid bonded continuous fiber nonwoven web comprising thermal point bonding;

Fig. 14 is a diagrammatic view of an apparatus for performing a process for producing a through-fluid bonded continuous fiber nonwoven web where vacuum forces are intermittently applied to the web;

Fig. 15 is a top view of an example vacuum diverter that may be used to block and/or reduce vacuum forces being applied a web;

Fig. 16 is a diagrammatic view of an apparatus for performing a process for producing a through-fluid bonded continuous fiber nonwoven web where vacuum forces are intermittently applied to the web and where hot and/or cold fluids are provided to the web;

Fig. 17 is a graph of the Martindale Average Abrasion Resistance Grade (y-axis) vs. DMA Initial Compression % (x-axis) showing continuous fiber present disclosure samples and carded related art samples values;

Fig. 18 is a perspective view of equipment for the Martindale Average Abrasion Resistance Grade Test herein; and

Fig. 19 is a grade scale for fuzz assessment in the Martindale Average Abrasion Resistance Grade Test herein.

DETAILED DESCRIPTION

Various non-limiting forms of the present disclosure will now be described to provide an overall understanding of the principles of the structure, function, manufacture, and use of the through-fluid bonded continuous fiber nonwoven webs disclosed herein. One or more examples of these non-limiting forms are illustrated in the accompanying drawings. Those of ordinary skill in the art will understand that the through-fluid bonded continuous fiber nonwoven webs described herein and illustrated in the accompanying drawings are non-limiting example forms and that the scope of the various non-limiting forms of the present disclosure are defined solely by the claims. The features illustrated or described in connection with one non-limiting form may be combined with the features of other non-limiting forms. Such modifications and variations are intended to be included within the scope of the present disclosure.

First, general characteristics, features, and/or components of example absorbent articles that may comprise the continuous fiber nonwoven web are discussed. Then, example methods of producing the continuous fiber nonwoven webs are discussed. Lastly, the properties of the produced continuous fiber nonwoven webs are discussed.

General Description of an Absorbent Article

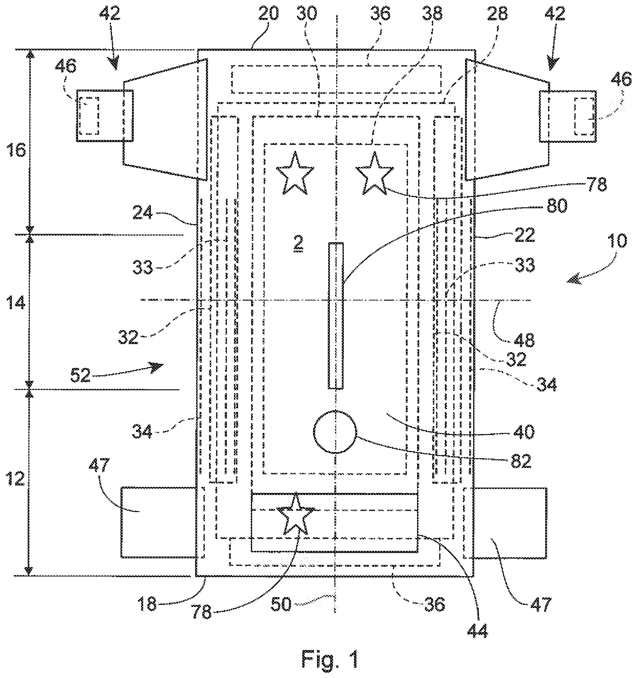

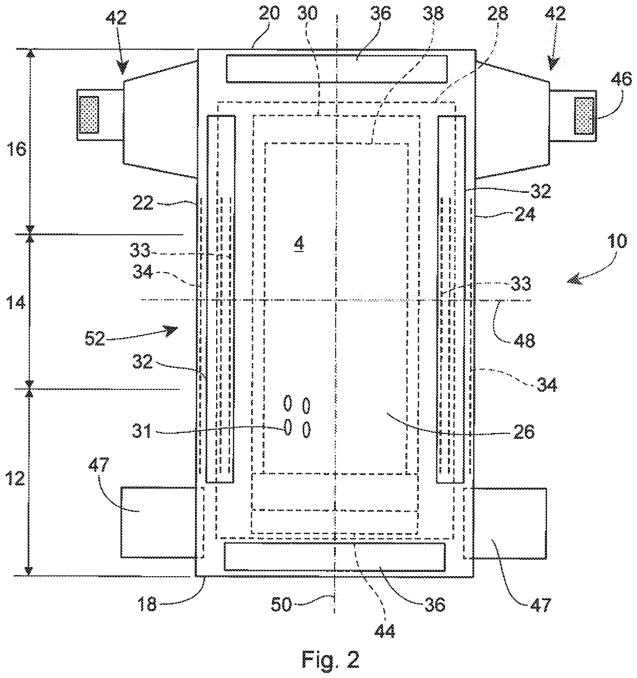

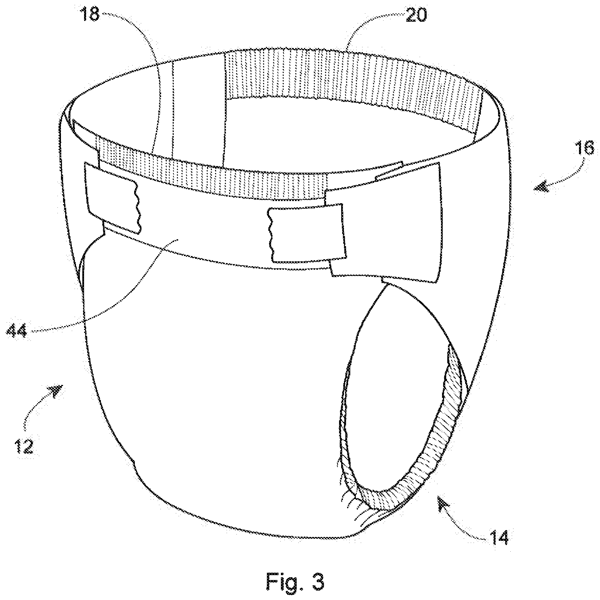

An example absorbent article 10 according to the present disclosure, shown in the form of a taped diaper, is represented in Figs. 1-3. Fig. 1 is a plan view of the example absorbent article 10, garment-facing surface 2 facing the viewer in a flat, laid-out state (i.e., no elastic contraction). Fig. 2 is a plan view of the example absorbent article 10 of Fig. 1, wearer-facing surface 4 facing the viewer in a flat, laid-out state. Fig. 3 is a front perspective view of the absorbent article 10 of Figs. 1 and 2 in a fastened configuration. The absorbent article 10 of Figs. 1-3 is shown for illustration purposes only as the present disclosure may be used for making a wide variety of diapers, including adult incontinence products, pants, or other absorbent articles, such as sanitary napkins and absorbent pads, for example.

The absorbent article 10 may comprise a front waist region 12, a crotch region 14, and a back waist region 16. The crotch region 14 may extend intermediate the front waist region 12 and the back waist region 16. The front wait region 12, the crotch region 14, and the back waist region 16 may each be 1/3 of the length of the absorbent article 10. The absorbent article 10 may comprise a front end edge 18, a back end edge 20 opposite to the front end edge 18, and longitudinally extending, transversely opposed side edges 22 and 24 defined by the chassis 52.

The absorbent article 10 may comprise a liquid permeable topsheet 26, a liquid impermeable backsheet 28, and an absorbent core 30 positioned at least partially intermediate the topsheet 26 and the backsheet 28. The absorbent article 10 may also comprise one or more pairs of barrier leg cuffs 32 with or without elastics 33, one or more pairs of leg elastics 34, one or more elastic waistbands 36, and/or one or more acquisition materials 38. The acquisition material or materials 38 may be positioned intermediate the topsheet 26 and the absorbent core 30. An outer cover material 40, such as a nonwoven material, may cover a garment-facing side of the backsheet 28. The absorbent article 10 may comprise back ears 42 in the back waist region 16. The back ears 42 may comprise fasteners 46 and may extend from the back waist region 16 of the absorbent article 10 and attach (using the fasteners 46) to the landing zone area or landing zone material 44 on a garment-facing portion of the front waist region 12 of the absorbent article 10. The absorbent article 10 may also have front ears 47 in the front waist region 12. The absorbent article 10 may have a central lateral (or transverse) axis 48 and a central longitudinal axis 50. The central lateral axis 48 extends perpendicular to the central longitudinal axis 50.

In other instances, the absorbent article may be in the form of a pant having permanent or refastenable side seams. Suitable refastenable seams are disclosed in U.S. Pat. Appl. Pub. No. 2014/0005020 and U.S. Pat. No. 9,421,137.

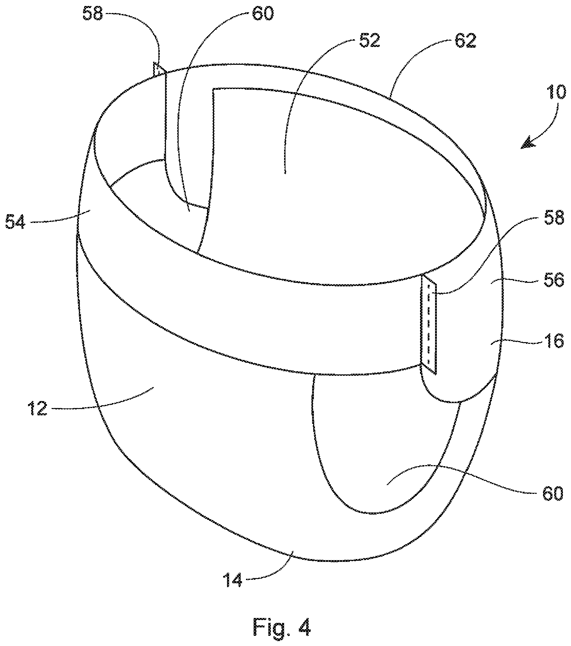

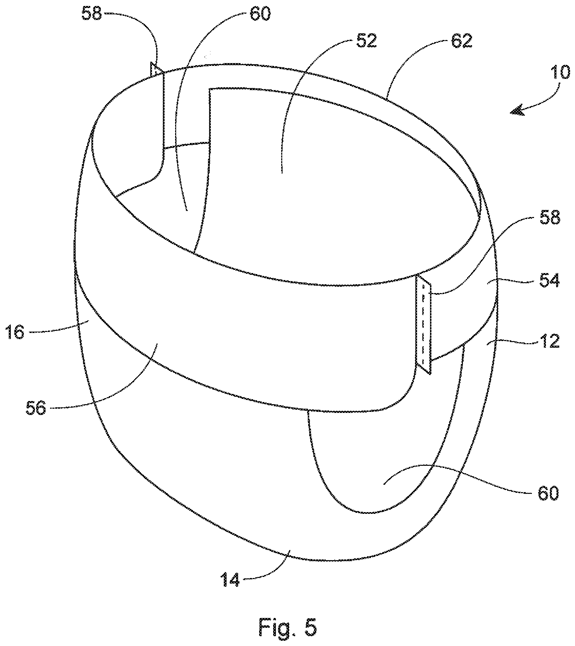

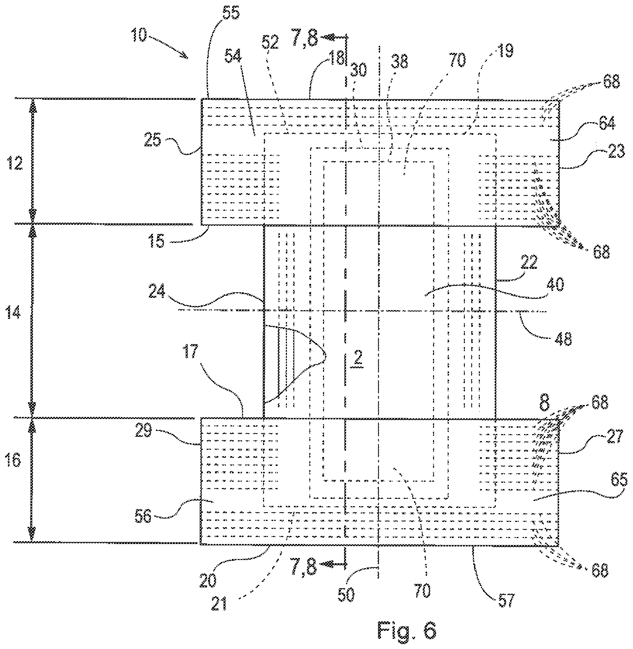

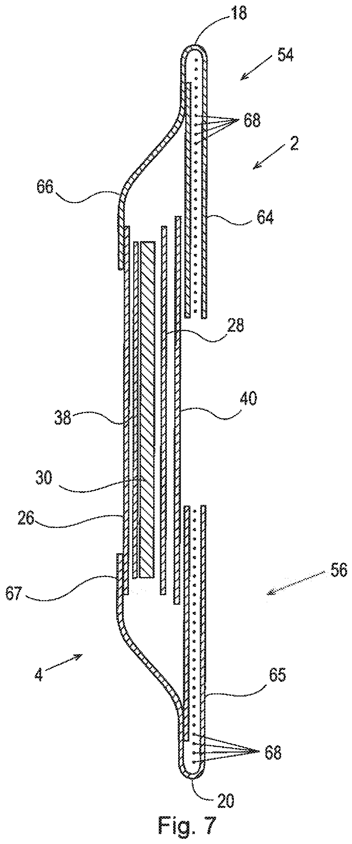

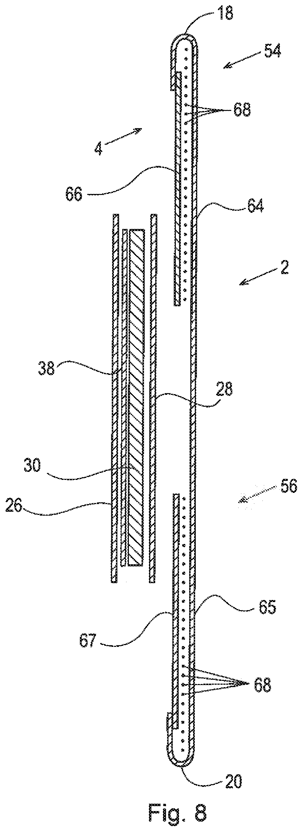

Referring to Figs. 4-8, an example absorbent article 10 in the form of a pant is illustrated. Fig. 4 is a front perspective view of the absorbent article 10. Fig. 5 is a rear perspective view of the absorbent article 10. Fig. 6 is a plan view of the absorbent article 10, laid flat, with the garment-facing surface facing the viewer. Elements of Fig. 4-8 having the same reference number as described above with respect to Figs. 1-3 may be the same element (e.g., absorbent core 30). Fig. 7 is an example cross-sectional view of the absorbent article taken about line 7-7 of Fig. 6. Fig. 8 is an example cross-sectional view of the absorbent article taken about line 8-8 of Fig. 6. Figs. 7 and 8 illustrate example forms of front and back belts 54, 56. The absorbent article 10 may have a front waist region 12, a crotch region 14, and a back waist region 16. Each of the regions 12, 14, and 16 may be 1/3 of the length of the absorbent article 10. The absorbent article 10 may have a chassis 52 (sometimes referred to as a central chassis or central panel) comprising a topsheet 26, a backsheet 28, and an absorbent core 30 disposed at least partially intermediate the topsheet 26 and the backsheet 28, and an optional acquisition material 38, similar to that as described above with respect to Figs. 1-3. The absorbent article 10 may comprise a front belt 54 in the front waist region 12 and a back belt 56 in the back waist region 16. The chassis 52 may be joined to a wearer-facing surface 4 of the front and back belts 54, 56 or to a garment-facing surface 2 of the belts 54, 56. Side edges 23 and 25 of the front belt 54 may be joined to side edges 27 and 29, respectively, of the back belt 56 to form two side seams 58. The side seams 58 may be any suitable seams known to those of skill in the art, such as butt seams or overlap seams, for example. When the side seams 58 are permanently formed or refastenably closed, the absorbent article 10 in the form of a pant has two leg openings 60 and a waist opening circumference 62. The side seams 58 may be permanently joined using adhesives or bonds, for example, or may be refastenably closed using hook and loop fasteners, for example.

Any nonwoven components of the absorbent articles may comprise the through-fluid bonded continuous fiber nonwoven webs of the present disclosure. In some instances, one or more nonwoven components may comprise the through-fluid continuous fiber nonwoven webs of the present disclosure, such as a topsheet and an outer cover nonwoven material, or a topsheet and a leg cuff, for example.

Belts

Referring to Figs. 7 and 8, the front and back belts 54 and 56 may comprise front and back inner belt layers 66 and 67 and front and back outer belt layers 64 and 65 having an elastomeric material (e.g., strands 68 or a film (which may be apertured)) disposed at least partially therebetween. The elastic elements 68 or the film may be relaxed (including being cut) to reduce elastic strain over the absorbent core 30 or, may alternatively, run continuously across the absorbent core 30. The elastics elements 68 may have uniform or variable spacing therebetween in any portion of the belts. The elastic elements 68 may also be pre-strained the same amount or different amounts. The front and/or back belts 54 and 56 may have one or more elastic element free zones 70 where the chassis 52 overlaps the belts 54, 56. In other instances, at least some of the elastic elements 68 may extend continuously across the chassis 52.

The front and back inner belt layers 66, 67 and the front and back outer belt layers 64, 65 may be joined using adhesives, heat bonds, pressure bonds or thermoplastic bonds. Various suitable belt layer configurations can be found in U.S. Pat. Appl. Pub. No. 2013/0211363.

Front and back belt end edges 55 and 57 may extend longitudinally beyond the front and back chassis end edges 19 and 21 (as shown in Fig. 6) or they may be co-terminus. The front and back belt side edges 23, 25, 27, and 29 may extend laterally beyond the chassis side edges 22 and 24. The front and back belts 54 and 56 may be continuous (i.e., having at least one layer that is continuous) from belt side edge to belt side edge (e.g., the transverse distances from 23 to 25 and from 27 to 29). Alternatively, the front and back belts 54 and 56 may be discontinuous from belt side edge to belt side edge (e.g., the transverse distances from 23 to 25 and 27 to 29), such that they are discrete.

As disclosed in U.S. Pat. No. 7,901,393, the longitudinal length (along the central longitudinal axis 50) of the back belt 56 may be greater than the longitudinal length of the front belt 54, and this may be particularly useful for increased buttocks coverage when the back belt 56 has a greater longitudinal length versus the front belt 54 adjacent to or immediately adjacent to the side seams 58.

The front outer belt layer 64 and the back outer belt layer 65 may be separated from each other, such that the layers are discrete or, alternatively, these layers may be continuous, such that a layer runs continuously from the front belt end edge 55 to the back belt end edge 57. This may also be true for the front and back inner belt layers 66 and 67 - that is, they may also be longitudinally discrete or continuous. Further, the front and back outer belt layers 64 and 65 may be longitudinally continuous while the front and back inner belt layers 66 and 67 are longitudinally discrete, such that a gap is formed between them - a gap between the front and back inner and outer belt layers 64, 65, 66, and 67 is shown in Fig. 7 and a gap between the front and back inner belt layers 66 and 67 is shown in Fig. 8.

The front and back belts 54 and 56 may include slits, holes, and/or perforations providing increased breathability, softness, and a garment-like texture. Underwear-like appearance can be enhanced by substantially aligning the waist and leg edges at the side seams 58 (see Figs. 4 and 5).

The front and back belts 54 and 56 may comprise graphics (see e.g., 78 of Fig. 1). The graphics may extend substantially around the entire circumference of the absorbent article 10 and may be disposed across side seams 58 and/or across proximal front and back belt seams 15 and 17; or, alternatively, adjacent to the seams 58, 15, and 17 in the manner described in U.S. Pat. No. 9,498, 389 to create a more underwear-like article. The graphics may also be discontinuous.

Alternatively, instead of attaching belts 54 and 56 to the chassis 52 to form a pant, discrete side panels may be attached to side edges of the chassis 22 and 24. Suitable forms of pants comprising discrete side panels are disclosed in U.S. Pat. Nos. 6,645,190; 8,747,379; 8,372,052; 8,361,048; 6,761,711; 6,817,994; 8,007,485; 7,862,550; 6,969,377; 7,497,851; 6,849,067; 6,893,426; 6,953,452; 6,840,928; 8,579,876; 7,682,349; 7,156,833; and 7,201,744.

The belts may comprise one of more of the through-fluid bonded continuous fiber nonwoven webs disclosed herein.

## Top sheet

The topsheet 26 is the part of the absorbent article 10 that is in contact with the wearer's skin. The topsheet 26 may be joined to portions of the backsheet 28, the absorbent core 30, the barrier leg cuffs 32, and/or any other layers as is known to those of ordinary skill in the art. The topsheet 26 may be compliant, soft-feeling, and non-irritating to the wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may have one or more layers. The topsheet may be apertured (Fig. 2, element 31), may have any suitable three-dimensional features, and/or may have a plurality of embossments (e.g., a bond pattern). The topsheet may be apertured by overbonding a material and then rupturing the overbonds through ring rolling, such as disclosed in U.S. Patent No. 5,628,097, to Benson et al., issued on May 13, 1997 and disclosed in U.S. Pat. Appl. Publication No. US 2016/0136014 to Arora et al. Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet. The topsheet may comprise one or more of the through-fluid bonded continuous fiber nonwoven webs disclosed herein.

## Backsheet

The backsheet 28 is generally that portion of the absorbent article 10 positioned proximate to the garment-facing surface of the absorbent core 30. The backsheet 28 may be joined to portions of the topsheet 26, the outer cover material 40, the absorbent core 30, and/or any other layers of the absorbent article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials which permit vapors to escape from the absorbent article, while still preventing, or at least inhibiting, bodily exudates from passing through the backsheet.

## Outer Cover Material

The outer cover material (sometimes referred to as a backsheet nonwoven) 40 may comprise one or more nonwoven materials joined to the backsheet 28 and that covers the backsheet 28. The outer cover material 40 forms at least a portion of the garment-facing surface 2 of the absorbent article 10 and effectively "covers" the backsheet 28 so that film is not present on the garment-facing surface 2. The outer cover material 40 may comprise a bond pattern, apertures, and/or three-dimensional features. The outer cover material may comprise one or more of the through-fluid bonded continuous fiber nonwoven webs disclosed herein.

## Absorbent Core

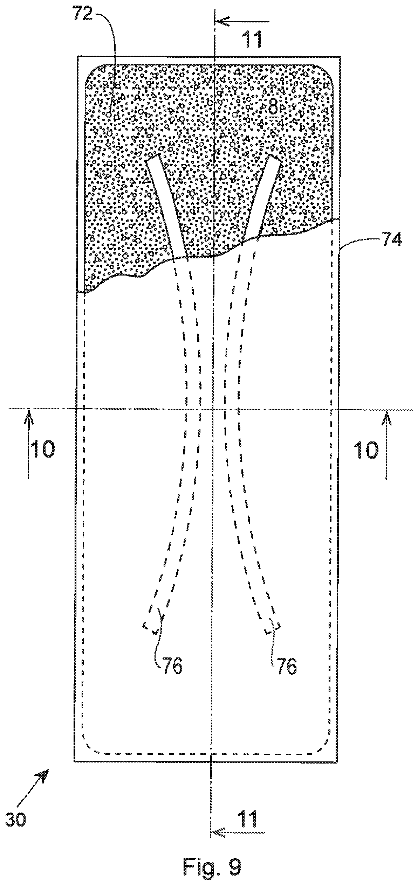

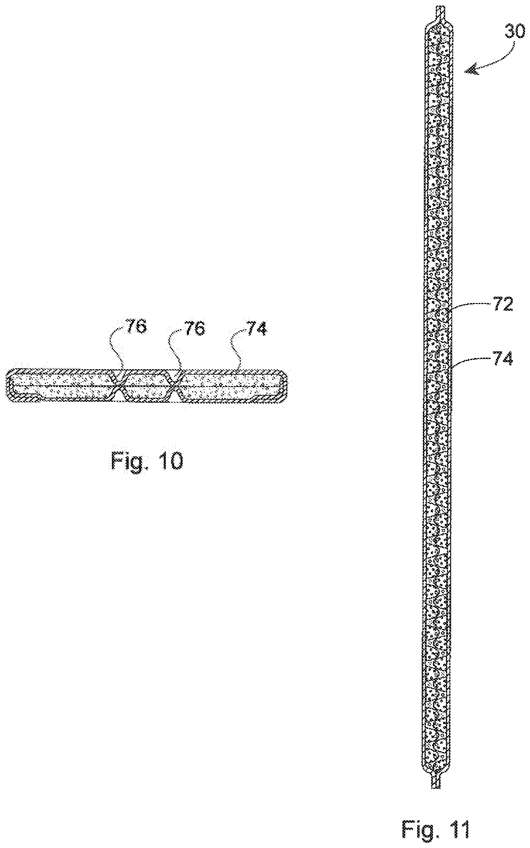

As used herein, the term "absorbent core" 30 refers to the component of the absorbent article 10 having the most absorbent capacity and that comprises an absorbent material. Referring to Figs. 9-11, in some instances, absorbent material 72 may be positioned within a core bag or a core wrap 74. The absorbent material may be profiled or not profiled, depending on the specific absorbent article. The absorbent core 30 may comprise, consist essentially of, or consist of, a core wrap, absorbent material 72, and glue enclosed within the core wrap. The absorbent material may comprise superabsorbent polymers, a mixture of superabsorbent polymers and air felt, only air felt, and/or a high internal phase emulsion foam. In some instances, the absorbent material may comprise at least 80%, at least 85%, at least 90%, at least 95%, at least 99%, or up to 100% superabsorbent polymers, by weight of the absorbent material. In such instances, the absorbent material may be free of air felt, or at least mostly free of air felt. The absorbent core periphery, which may be the periphery of the core wrap, may define any suitable shape, such as rectangular "T," "Y," "hour-glass," or "dog-bone" shaped, for example. An absorbent core periphery having a generally "dog bone" or "hour-glass" shape may taper along its width towards the crotch region 14 of the absorbent article 10.

Referring to Figs. 9-11, the absorbent core 30 may have areas having little or no absorbent material 72, where a wearer-facing surface of the core bag 74 may be joined to a garment-facing surface of the core bag 74. These areas having little or no absorbent material and may be referred to as "channels" 76. These channels can embody any suitable shapes and any suitable number of channels may be provided. In other instances, the absorbent core may be embossed to create the impression of channels. The absorbent core in Figs. 9-11 is merely an example absorbent core. Many other absorbent cores with or without channels are also within the scope of the present disclosure.

The core bag may comprise one or more of the through-fluid bonded continuous fiber nonwoven webs disclosed herein.

## Barrier Leg Cuffs/Leg Elastics

Referring to Figs. 1 and 2, for example, the absorbent article 10 may comprise one or more pairs of barrier leg cuffs 32 and one or more pairs of leg elastics 34. The barrier leg cuffs 32 may be positioned laterally inboard of leg elastics 34. Each barrier leg cuff 32 may be formed by a piece of material which is bonded to the absorbent article 10 so it can extend upwards from a wearer-facing surface 4 of the absorbent article 10 and provide improved containment of body exudates approximately at the junction of the torso and legs of the wearer. The barrier leg cuffs 32 are delimited by a proximal edge joined directly or indirectly to the top sheet and/or the backsheet and a free terminal edge, which is intended to contact and form a seal with the wearer's skin. The barrier leg cuffs 32 may extend at least partially between the front end edge 18 and the back end edge 20 of the absorbent article 10 on opposite sides of the central longitudinal axis 50 and may be at least present in the crotch region 14. The barrier leg cuffs 32 may each comprise one or more elastics 33 (e.g., elastic strands or strips) near or at the free terminal edge. These elastics 33 cause the barrier leg cuffs 32 to help form a seal around the legs and torso of a wearer. The leg elastics 34 extend at least partially between the front end edge 18 and the back end edge 20. The leg elastics 34 essentially cause portions of the absorbent article 10 proximate to the chassis side edges 22, 24 to help form a seal around the legs of the wearer. The leg elastics 34 may extend at least within the crotch region 14.

The barrier leg cuffs/leg elastics may comprise one or more of the through-fluid bonded continuous fiber nonwoven webs disclosed herein.

## Elastic Waistband

Referring to Figs. 1 and 2, the absorbent article 10 may comprise one or more elastic waistbands 36. The elastic waistbands 36 may be positioned on the garment-facing surface 2 or the wearer-facing surface 4. As an example, a first elastic waistband 36 may be present in the front waist region 12 near the front belt end edge 18 and a second elastic waistband 36 may be present in the back waist region 16 near the back end edge 20. The elastic waistbands 36 may aid in sealing the absorbent article 10 around a waist of a wearer and at least inhibiting bodily exudates from escaping the absorbent article 10 through the waist opening circumference. In some instances, an elastic waistband may fully surround the waist opening circumference of an absorbent article.

The waistbands may comprise one or more of the through-fluid bonded continuous fiber nonwoven webs disclosed herein.

## Acquisition Materials

Referring to Figs. 1, 2, 7, and 8, one or more acquisition materials 38 may be present at least partially intermediate the topsheet 26 and the absorbent core 30. The acquisition materials 38 are typically hydrophilic materials that provide significant wicking of bodily exudates. These materials may dewater the topsheet 26 and quickly move bodily exudates into the absorbent core 30. The acquisition materials 38 may comprise one or more nonwoven materials, foams, cellulosic materials, cross-linked cellulosic materials, air laid cellulosic nonwoven materials, spunlace materials, or combinations thereof, for example. In some instances, portions of the acquisition materials 38 may extend through portions of the topsheet 26, portions of the topsheet 26 may extend through portions of the acquisition materials 38, and/or the topsheet 26 may be nested with the acquisition materials 38. Typically, an acquisition material 38 may have a width and length that are smaller than the width and length of the topsheet 26. The acquisition material may be a secondary topsheet in the feminine pad context. The acquisition material may have one or more channels as described above with reference to the absorbent core 30 (including the embossed version). The channels in the acquisition material may align or not align with channels in the absorbent core 30. In an example, a first acquisition material may comprise a nonwoven material and as second acquisition material may comprise a cross-linked cellulosic material.

The acquisition material may comprise one or more of the through-fluid bonded continuous fiber nonwoven webs disclosed herein.

Landing Zone

Referring to Figs. 1 and 2, the absorbent article 10 may have a landing zone area 44 that is formed in a portion of the garment-facing surface 2 of the outer cover material 40. The landing zone area 44 may be in the back waist region 16 if the absorbent article 10 fastens from front to back or may be in the front waist region 12 if the absorbent article 10 fastens back to front. In some instances, the landing zone 44 may be or may comprise one or more discrete nonwoven materials that are attached to a portion of the outer cover material 40 in the front waist region 12 or the back waist region 16 depending upon whether the absorbent article fastens in the front or the back. In essence, the landing zone 44 is configured to receive the fasteners 46 and may comprise, for example, a plurality of loops configured to be engaged with, a plurality of hooks on the fasteners 46, or *vice versa*.

The landing zone may comprise one or more of the through-fluid bonded continuous fiber nonwoven webs disclosed herein.

Wetness Indicator/Graphics

Referring to Fig. 1, the absorbent articles 10 of the present disclosure may comprise graphics 78 and/or wetness indicators 80 that are visible from the garment-facing surface 2. The graphics 78 may be printed on the landing zone 40, the backsheet 28, and/or at other locations. The wetness indicators 80 are typically applied to the absorbent core facing side of the backsheet 28, so that they can be contacted by bodily exudates within the absorbent core 30. In some instances, the wetness indicators 80 may form portions of the graphics 78. For example, a wetness indicator may appear or disappear and create/remove a character within some graphics. In other instances, the wetness indicators 80 may coordinate (e.g., same design, same pattern, same color) or not coordinate with the graphics 78.

Front and Back Ears

Referring to Figs. 1 and 2, as referenced above, the absorbent article 10 may have front and/or back ears 47, 42 in a taped diaper context. Only one set of ears may be required in most taped diapers. The single set of ears may comprise fasteners 46 configured to engage the landing zone or landing zone area 44. If two sets of ears are provided, in most instances, only one set of the ears may have fasteners 46, with the other set being free of fasteners. The ears, or portions thereof, may be elastic or may have elastic panels. In an example, an elastic film or elastic strands may be positioned intermediate a first nonwoven material and a second nonwoven material. The elastic film may or may not be apertured. The ears may be shaped. The ears may be integral (e.g., extension of the outer cover material 40, the backsheet 28, and/or the topsheet 26) or may be discrete components attached to a chassis 52 of the absorbent article on a wearer-facing surface 4, on the garment-facing surface 2, or intermediate the two surfaces 4, 2.

The front and back ears may comprise one or more of the through-fluid bonded continuous fiber nonwoven webs disclosed herein.

Sensors

Referring again to Fig. 1, the absorbent articles of the present disclosure may comprise a sensor system 82 for monitoring changes within the absorbent article 10. The sensor system 82 may be discrete from or integral with the absorbent article 10. The absorbent article 10 may comprise sensors that can sense various aspects of the absorbent article 10 associated with insults of bodily exudates such as urine and/or BM (e.g., the sensor system 82 may sense variations in temperature, humidity, presence of ammonia or urea, various vapor components of the exudates (urine and feces), changes in moisture vapor transmission through the absorbent articles garment-facing layer, changes in translucence of the garment-facing layer, and/or color changes through the garment-facing layer). Additionally, the sensor system 82 may sense components of urine, such as ammonia or urea and/or byproducts resulting from reactions of these components with the absorbent article 10. The sensor system 82 may sense byproducts that are produced when urine mixes with other components of the absorbent article 10 (e.g., adhesives, agm). The components or byproducts being sensed may be present as vapors that may pass through the garment-facing layer. It may also be desirable to place reactants in the absorbent article that change state (e.g. color, temperature) or create a measurable byproduct when mixed with urine or BM. The sensor system 82 may also sense changes in pH, pressure, odor, the presence of gas, blood, a chemical marker or a biological marker or combinations thereof. The sensor system 82 may have a component on or proximate to the absorbent article that transmits a signal to a receiver more distal from the absorbent article, such as an iPhone, for example. The receiver may output a result to communicate to the caregiver a condition of the absorbent article 10. In other instances, a receiver may not be provided, but instead the condition of the absorbent article 10 may be visually or audibly apparent from the sensor on the absorbent article.

Packages

The absorbent articles of the present disclosure may be placed into packages. The packages may comprise polymeric films and/or other materials, such as the through-fluid bonded continuous fiber nonwoven webs disclosed herein. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

Sanitary Napkin/Liners

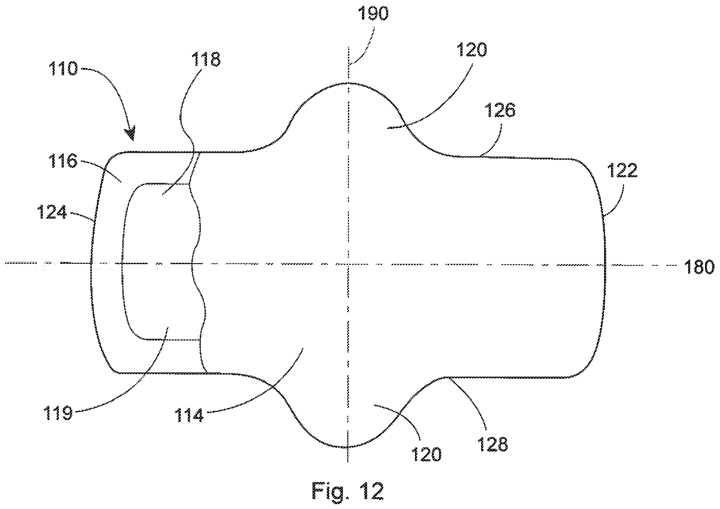

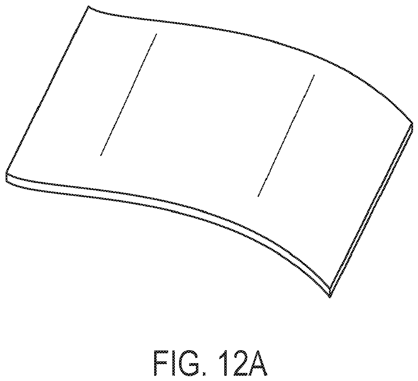

Referring to Fig. 12, an absorbent article of the present disclosure may be a sanitary napkin 110. The sanitary napkin 110 may comprise a liquid permeable topsheet 114, a liquid impermeable, or substantially liquid impermeable, backsheet 116, and an absorbent core 118. The liquid impermeable backsheet 116 may or may not be vapor permeable. The absorbent core 118 may have any or all of the features described herein with respect to the absorbent core 30 and, in some forms, may have a secondary topsheet 119 (STS) instead of the acquisition materials disclosed above. The STS 119 may comprise one or more channels, as described above (including the embossed version). In some forms, channels in the STS 119 may be aligned with channels in the absorbent core 118. The sanitary napkin 110 may also comprise wings 120 extending outwardly with respect to a longitudinal axis 180 of the sanitary napkin 110. The sanitary napkin 110 may also comprise a lateral axis 190. The wings 120 may be joined to the topsheet 114, the backsheet 116, and/or the absorbent core 118. The sanitary napkin 110 may also comprise a front edge 122, a back edge 124 longitudinally opposing the front edge 122, a first side edge 126, and a second side edge 128 longitudinally opposing the first side edge 126. The longitudinal axis 180 may extend from a midpoint of the front edge 122 to a midpoint of the back edge 124. The lateral axis 190 may extend from a midpoint of the first side edge 128 to a midpoint of the second side edge 128. The sanitary napkin 110 may also be provided with additional features commonly found in sanitary napkins as is known in the art.
The topsheet, secondary topsheet, wings, or any other nonwoven components of the sanitary napkin or a liner may comprise one or more of the through-fluid bonded continuous fiber nonwoven webs disclosed herein.

Wipes

The through-fluid bonded continuous fiber nonwoven webs of the present disclosure may form at least portions of, or all of, wipes (see Fig. 12A), such as wet wipes, dry wipes, makeup removal wipes, cleaning wipes, and/or dusting wipes, for example. Cleaning wipes and dusting wipes include products sold under the Swiffer® Brand that are manufactured by The Procter & Gamble Company of Cincinnati, Ohio.

Continuous Fiber Composition

The continuous fibers of the nonwoven webs of the present disclosure may comprise multi-constituent fibers, such as bicomponent fibers or tri-component fibers, for example, mono-component fibers, and/or other fiber types. Multi-constituent fibers, as used herein, means fibers comprising more than one chemical species or material (i.e., multi-component fibers). Bicomponent fibers are used in the present disclosure merely as an example of multi-constituent fibers. The fibers may have round, triangular, tri-lobal, or otherwise shaped cross-sections, for example. It may be desirable to have fibers comprising more than one polymer component, such as bicomponent fibers. Often, these two polymer components have different melting temperatures, viscosities, glass transition temperatures, and/or crystallization rates. As the bicomponent fibers cool after formation, one polymer component may solidify and/or shrink at a faster rate than the other polymer component, deforming the fiber, causing increased bending in the fiber when tension on the fiber is relieved, and thereby causing what is known as "crimp" in the fibers. Crimp of the fibers aids in the softness and loft of a nonwoven web, which is consumer desirable. Examples of bicomponent fibers may comprise a first polymer component having a first melting temperature and a second polymer component having a second melting temperature. The first melting temperature of the first polymer component may be about 10 degrees C to about 180 degrees C, or about 30 degrees C to about 150 degrees C, different than the second melting temperature of the second polymer component, thereby causing crimping of the fibers during cooling, specifically reciting all 0.1 degree C increments within the specified ranges and all ranges formed therein or thereby. The first and second melting temperatures may differ by at least 10 degrees C, at least 25 degrees, at least 40 degrees, at least 50 degrees, at least 75 degrees, at least 100 degrees C, at least 125 degrees C, at least 150 degrees C, but all less than 180 degrees C, for example. As a further example, a first polymer component may comprise polypropylene and a second polymer component may comprise polyethylene. As yet another example, a first polymer component may comprise polyethylene and a second polymer component may comprise polyethylene terephthalate. As yet another example, a first polymer component may comprise polyethylene and a second polymer component may comprise polylactic acid. If tri-component fibers are used, at least one polymer component may have a different melting temperature (in the ranges specified above) than a melting temperature of at least one of the other two polymer components. The fibers may comprise natural resins, synthetic resins, recycled resins, polylactic acid resins, and/or bio-based resins. The fibers may be or may comprise continuous fibers or spun fibers. Carded staple fibers may also be within the scope of the methods of the present disclosure. The multi-constituent fibers, such as bicomponent fibers, may comprise sheath/core, side-by-side, islands in the sea, and/or eccentric configurations or may have other configurations.

Using thinner fibers may help through-fluid bonding intermediate continuous fiber nonwoven webs to produce continuous fiber nonwoven webs. For example, the continuous fibers may have a decitex in the range of about 0.5 to about 15, about 0.5 to about 10, about 0.5 to about 5, about 0.8 to about 4, about 0.8 to about 3, about 0.8 to about 2, about 0.8 to about 1.5, about 1 to about 1.4, about 1.1 to about 1.3, or about 1.2, specifically reciting all 0.1 decitex increments within the specified ranges and all ranges formed therein or thereby.

General Continuous Fiber Nonwoven Formation Process

Many nonwoven webs are made from melt-spinnable polymers and are produced using a spunbond process. The term "spunbond" refers to a process of forming a nonwoven web from thin continuous fibers produced by extruding molten polymers from orifices of a spinneret. The continuous fibers are drawn as they cool (e.g., by an aspirator, positioned below the spinneret, which longitudinally stretches and transversely attenuates the fibers) and are randomly laid on a moving porous member, such as a moving porous belt, such that the continuous fibers form an intermediate continuous fiber nonwoven web. The intermediate continuous fiber nonwoven web is subsequently bonded using one of several known techniques, such as thermal point bonding or air through bonding, for example, to form the nonwoven web. Spunbonding processes, however, result in low loft and softness in produced nonwoven webs due to the heavy thermal point bonding and reduced ability for the fibers to crimp on the moving porous member.

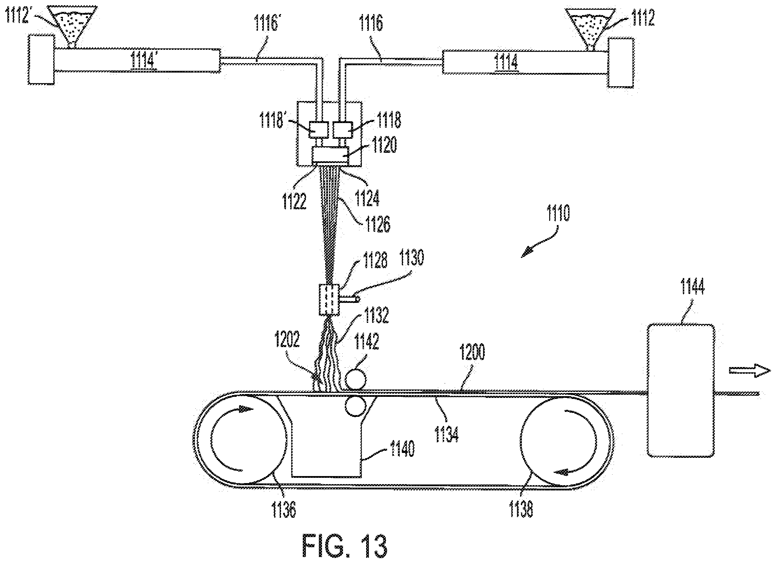

Fig. 13 diagrammatically illustrates an example apparatus 1110 for producing continuous fiber nonwoven webs. The apparatus 1110 may comprise a hopper 1112 into which pellets of a solid polymer may be placed. The polymer may be fed from the hopper 1112 to a screw extruder 1114 that melts the polymer pellets. The molten polymer may flow through a heated pipe 1116 to a metering pump 1118 that in turn feeds the polymer stream to a suitable spin pack 1120. The spin pack 1120 may comprise a spinneret 1122 defining a plurality of orifices 1124 that shape the fibers extruded therethrough. The orifices may be any suitable shape, such as round, for example. If bicomponent fibers are desired, another hopper 1112', another screw extruder 1114', another heated pipe 1116', and another metering pump 1118' may be included to feed a second polymer to the spinneret 1122. The second polymer may be the same as or different than the first polymer. In some instances, the second polymer may be a different material and may have a different melting temperature as the first polymer as discussed herein. This difference in melting temperature allows formed bicomponent fibers to crimp on the moving porous member as discussed herein. More than two polymer feed systems may also be included if a 3 or more polymer components are desired.

Referring again to Fig. 13, an array of continuous fiber strands 1126 may exit the spinneret 1122 of the spin pack 1120 and may be pulled downward by a drawing unit or aspirator 1128, which may be fed by a fluid, such as compressed air or steam, from a conduit or other fluid source 1130. Specifically, the aspirator 1128 uses fluid pressure or air pressure to form a fluid flow or air flow directed generally downward toward the moving porous member, which creates a downward fluid drag or air drag on the continuous fibers, thereby increasing the velocity of the portion of the continuous fiber strands in and below the aspirator relative to the velocity of the portion of the continuous fibers above the aspirator. The downward drawing of the continuous fibers longitudinally stretches and transversely attenuates the continuous fibers. The aspirator 1128 may be, for example, of the gun type or of the slot type, extending across the full width of the continuous fiber array, i.e., in the direction corresponding to a width of the intermediate nonwoven web to be formed by the continuous fibers. The area between the spinneret 1122 and the aspirator 1128 may be open to ambient air (open system) as illustrated or closed to ambient air (closed system).

The aspirator 1128 delivers the attenuated continuous fiber strands 1132 onto a moving porous member 1134, such as a screen-type forming belt, which may be supported and driven by rolls 1136 and 1138 or other mechanisms. A suction box 1140 may provide a negative fluid pressure to the moving porous member 1134 and the intermediate continuous fiber nonwoven web on the moving porous member 1134. For example, the suction box 1140 may be connected to a fan to pull room air (at the ambient temperature) through the moving porous member 1134, causing the continuous fibers 1132 to form an intermediate continuous fiber nonwoven web 1200 on moving porous member 1134. The intermediate continuous fiber web 1200 may pass through a thermal point bonding unit 1142 or a through-air fluid bonding unit to provide the web 1200 with structural integrity as it travels downstream of the first location 1202. The intermediate continuous fiber nonwoven web 1200 may then be conveyed on the moving porous member 1134 or other conveyer or belt into a through-fluid bonding oven 1144.

The moving porous member 1134 may be a structured forming belt with a resin disposed thereon, as described in U.S. Pat. No. 10,190,244, issued on January 29, 2019, to Ashraf et al. The moving porous member 134 may be a SupraStat 3601 belt from Albany International Corp.

Example materials are contemplated where the first and/or second polymers of the bicomponent continuous fibers comprise additives in addition to their constituent chemistry. For example, suitable additives comprise additives for coloration, antistatic properties, lubrication, softness, hydrophilicity, hydrophobicity, and the like, and combinations thereof. Silky additives may also be used such as an amide family additive, a steric acid, a functionalized siloxane, and/or a wax, for example. These additives, for example titanium dioxide for coloration, may generally be present in an amount less than about 5 weight percent and more typically less than about 2 weight percent or less of the total weight of the fibers.

Example Methods of Producing Through-Fluid Bonded Continuous Fiber Nonwoven Webs of the Present Disclosure

In order to allow better continuous fiber crimping on the moving porous member 134, and thereby promote improved softness, loft, and fiber reorientation, the present inventors have determined that applying variable or intermittent vacuum forces to the intermediate continuous fiber nonwoven in different zones (machine direction zones or cross-machine direction zones) of the moving porous member 1134 is desired. The variable or intermittent vacuum forces may be on/off. Alternatively, the variable or intermittent vacuum forces may be a first vacuum force and a second smaller vacuum force. In any event, when the vacuum forces applied to the intermediate continuous fiber nonwoven web are turned off or reduced, the web is allowed to relax or partially relax, leading to continuous fiber reorientation occurring and nonwoven web thickening in the z-direction. Turning the vacuum force on/off, or first vacuum force/second smaller vacuum force multiple times, provides improved benefits for nonwoven web stability and strength from fiber crimping and fiber reorientation before through-fluid bonding. These variable or intermittent vacuum supplying steps provide soft and lofty intermediate continuous fiber nonwoven webs with improved continuous fiber reorientation for better structural integrity. By improved continuous fiber reorientation, it is meant that the continuous fibers are more entangled with each other and have improved continuous fiber crimping. In the off vacuum zones, a positive fluid pressure may be applied to the web to aid in providing loft and softness to the web.

Vacuum forces may be quantified by measuring the vacuum air velocity with and anemometer, such as Extech CFM/CMM Thermo-Anemometer (Part # 407113), for example. To measure the air velocity, the Thermo-Anemometer is placed above and in contact with the moving porous member in the absence of the nonwoven web and with the moving porous member stopped. The vacuum forces and their corresponding velocities may depend on a number of factors, such as vacuum zone length or size, moving porous member speed (when running), fiber composition, and/or basis weight. Air velocities may be high enough to substantially collapse the lofted structure but allow it to transfer smoothly across the vacuum zone without breaking apart. For example, vacuum air velocities may be as high as 10 m/s, as high as 5 m/s, as high as 4 m/s, as high as 3 m/s, as high as 2 m/s, or as high as 1 m/s. The machine direction length of the vacuum zones may depend on a number of factors, such as vacuum air velocity, moving porous member speed (when running), fiber composition, and/or basis weight. Air vacuum zones may be large enough to substantially collapse the lofted web structure, but still allow the lofted web structure to transfer smoothly across the vacuum zone without breaking apart. For example, air vacuum zone machine direction lengths may be as high as 20 cm, as high as 10 cm, as high as 5 cm, as high as 2.5 cm or as high as 1 cm, for example.

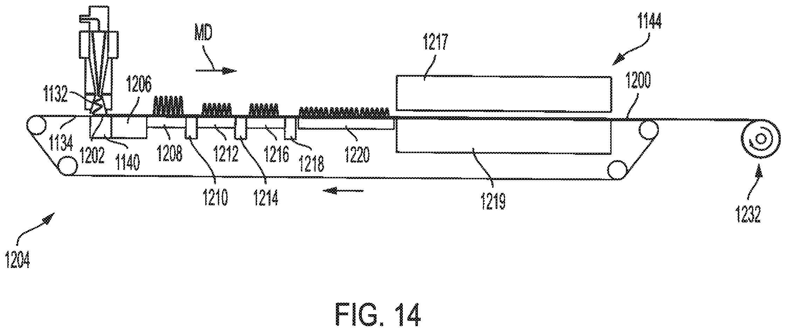

Referring to Fig. 14, an apparatus 1204 for producing a continuous fiber nonwoven web 1200 is illustrated. The general process of creating continuous fiber strands 1132 and depositing them on a moving porous member 1134 is described above with respect to Fig. 13 and will not be repeated here for brevity. The continuous fibers may comprise bicomponent fibers having a first polymer and a second polymer. The first polymer may have a first melting temperature and the second polymer may have a second melting temperature. The first melting temperature may be different than the second melting temperature in the range of about 10 degrees to about 180 degrees, or about 30 degrees to about 150 degrees, including the other ranges specified herein. This difference in melting temperatures of the polymers causes the continuous fibers to crimp during fiber cooling. Crimping promotes loft, softness, and fiber reorientation in a nonwoven web, which are all desirable properties. The more the continuous fibers are allowed to crimp on the moving porous member 1134 during cooling, the better loft, softness, and fiber reorientation the nonwoven web may achieve.

As discussed with respect to Fig. 13, the continuous fiber strands 1132 are deposited on the moving porous member 1134 at a first location 1202 to form an intermediate continuous fiber nonwoven web 1200. The intermediate continuous fiber nonwoven web 200 is then conveyed by the moving porous member 1134 downstream (i.e., in the machine direction or MD) toward a through-fluid bonding oven 1144. This same concept applies to Fig. 2, as indicated by the reference numbers in Fig. 14. Once the web 1200 is conveyed downstream of the vacuum box 1140, it may experience variable or intermittent vacuum forces prior to being conveyed into the through-fluid bonding oven 1144. These variable or intermittent vacuum forces applied to the web may occur without the addition of any more continuous fibers on the moving porous member 1134 and without any additional heat being applied. The moving porous member 1134 may be conveyed on rollers, for example. It is noted that any of the "moving porous members" disclosed herein may have sections or portions that are not porous, but at least some sections or portions of the moving porous members are able to have a fluid flow therethrough.

As an example, the web 1200 may be conveyed through a first zone 1206 downstream of the first location 1202 and downstream of the vacuum box 1140, a second zone 1208 downstream of the first zone 1206, a third zone 1210 downstream of the second zone 1208, and a fourth zone 1212 downstream of the third zone 1210 prior to being conveyed into the through-fluid bonding oven 1144. In some instances, the web 1200 may also be conveyed through a fifth zone 1214 downstream of the fourth zone 1212 and a sixth zone 1216 downstream of the fifth zone 1214 before being conveyed into the through-fluid bonding oven 1144. In still other instances, the web 1200 may also be conveyed through a seventh zone 1218 downstream of the sixth zone 1216 and an eighth zone 1220 downstream of the seventh zone 1218 prior to being conveyed into the through-fluid bonding oven 1144. Any suitable number of zones of intermittent or variable vacuum may be used within reason based on a footprint of a nonwoven manufacturing line. For example, 10 different zones may be used, 15 different zones may be used, or 20 different zones may be used. Further, the zones may not always be staggered as on/off or first vacuum force/second smaller vacuum force. Instead, multiple zones of no or reduced vacuum may be positioned together. For example, two zones of no or reduced vacuum may be positioned together with single zones of vacuum surrounding them.

Still referring to Fig. 14, a first vacuum force may be applied to the intermediate continuous fiber nonwoven web 1200 to the first zone 1206, the third zone 1210, and fifth zone 1214, and/or the seventh zone 1218 or more zones, if provided. A second vacuum force may be applied to the intermediate continuous fiber nonwoven web 1200 in the second zone 1208, the fourth zone 1212, the sixth zone 1216, and/or the eighth zone 1220 or more zones, if provided. The second vacuum force may be about zero, zero, or may merely be less than the first vacuum force. In any event, the intermittent or variable cycling of the vacuum force (whether on/off or merely reduced) applied to the intermediate continuous fiber nonwoven web 1200 allows the continuous fibers to relax, crimp, and reorient leading to improved loft, softness, and structural integrity.

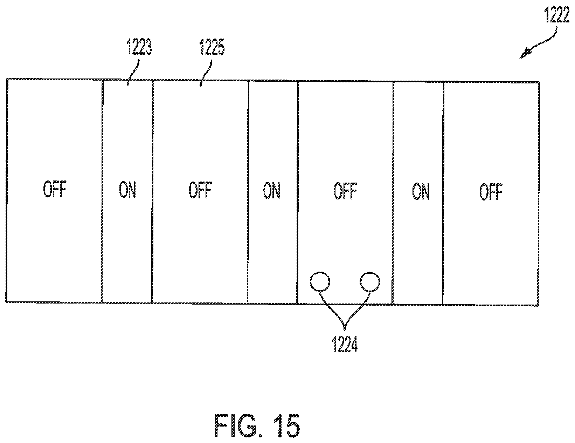

The various zones may all have the same machine directional lengths or may have different machine directional lengths. For example, the zones receiving vacuum forces may have shorter machine directional lengths than the zones not receiving vacuum forces or receiving reduced vacuum forces (see e.g., Fig. 15). In other instances, the zones receiving no vacuum forces or receiving reduced vacuum forces may have shorter machine directional lengths than the zones receiving vacuum forces. The various zones may all have the same cross-machine directional lengths or may have different cross-machine directional lengths. In some instances, a single zone may provide the web 1200 with a first vacuum force in a first area and a second different vacuum force in a second area. The second different vacuum force may be about zero or may merely be different.

Vacuum forces may be varied by only providing vacuum boxes under the individual zones of the moving porous member 1134 that are intended to receive the vacuum. In other instances, vacuum boxes may be provided under all of the zones, with some of the zones either receiving reduced vacuum or no vacuum. This may be accomplished by turning off the vacuum boxes or reducing the fluid being drawn by the vacuum boxes in the zones intended to receive reduced or no vacuum. Alternatively, vacuum may be drawn under the entire or most of the moving porous member 1134 and a vacuum diverter, such as a vacuum blocking plate 1222, for example, or other member may be positioned intermediate the vacuum sources or boxes and the moving porous member 1134 to eliminate or reduce vacuum from being applied to certain zones of the moving porous member 1134. Referring to Fig. 15, an example vacuum blocking plate 1222 is illustrated. The vacuum blocking plate 1222 may have cut out areas or material free-areas in which vacuum forces may pass ("ON" zones 1223) through to the intermediate continuous fiber nonwoven web 200. The vacuum blocking plate 1222 may have areas with material that block or reduce vacuum forces from passing ("OFF" zones 1225) through to the intermediate continuous fiber nonwoven web 1200. The OFF zones applying reduced vacuum forces may define apertures 1224, slots, or other holes to allow small vacuum forces to pass to the web 1200 to hold the web 1200 to the moving porous member 1134. As such, the OFF zones may be zones of no vacuum or zones of reduced vacuum.

The vacuum forces may not only be varied in the machine direction. Instead, the vacuum forces may be varied in the cross-machine direction and/or in the machine direction and the cross-machine direction.

Referring again to Fig. 14, the web 1200 may then be conveyed into the through-fluid bonding oven 1144. The through-fluid bonding oven 1144 may have multiple zones that heat the web or heat and/or cool the web to allow the continuous fibers to reorient and entangle. The continuous fiber nonwoven web 1200 may then be conveyed out of the through-fluid bonding oven 1144 to another process, such as winding 1232 or further bonding in another through-fluid bonding oven, for example.

The through-fluid bonding oven 1144 may take on various configurations, such as flat, omega shaped, single belt, or multiple belts, for example. More than one though-fluid bonding oven may be used. One example configuration is to have a hot fluid supply 1217, such as hot air, above the web 1200 and a hot fluid vacuum 1219 below the web 1200. Of course, this configuration could be reversed to provide loft to the web in a direction opposite to the vacuum forces applied during the continuous fiber laydown. The hot fluid may be recycled in the through-fluid bonding oven 1144. The hot fluid may travel through the through-fluid bonding oven 1144 at a flow rate in the range of about 0.5 m/s to about 5 m/s and at a temperature in the range of about 10 degrees C to about 280 degrees C, for example. In some instances, it may be desirable to also have cooling within the through-fluid oven to set the fiber-to-fiber bonding. The through-fluid bonding oven belts or porous support members may be preheated in the range of about 5 degrees C to about 130 degrees C or about 50 degrees C to about 130 degrees C for improved efficiency in bonding.

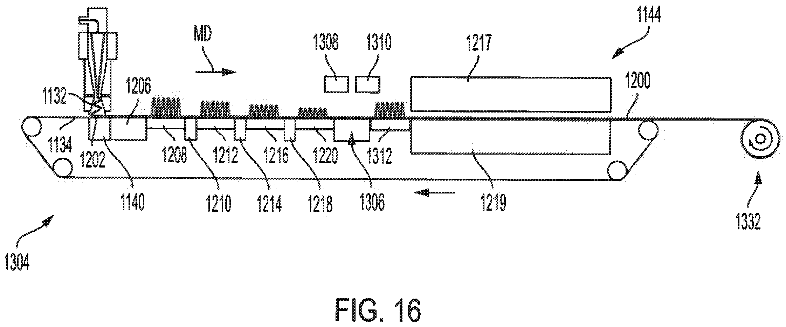

Referring to Fig. 16, an apparatus 1304 for producing a continuous fiber nonwoven web 1200 is illustrated. The apparatus 1304 is similar to the apparatus 1204 of Fig. 14, but also shows additional process steps. In the apparatus 1304, the intermediate web of continuous fibers 1200 is deposited on the moving porous member 1134 in the same or a similar fashion as described with respect to Figs. 13 and 14. The web 1200 may be conveyed through the various vacuum zones as discussed with respect to Fig. 14. The various zones of Fig. 16 are labeled the same as Fig. 14 and perform the same or a similar function. The vacuum blocking plate or vacuum diverter of Fig. 15 may also be used in the various zones, much like the example apparatus 1204 of Fig. 14. The apparatus 1304, however, applies additional transformations to the web 1200 prior to the web 1200 entering the through-fluid bonding oven 1144 and after the intermittingly varying the vacuum force steps.

First, the apparatus 1304 may comprise a temperature variation zone 1306. Heating 1308 and/or cooling 1310 may be applied to the web 1200 in the temperature variation zone 1306. The heat may be in the form of a heated fluid, such as hot air having a temperature in the range of about 30 degrees C to about 130 degrees C, for example. An air knife may be an appropriate tool to provide the heat. The heat may be applied to the web 1200 while the web 1200 is under a vacuum force, a reduced vacuum force, or no vacuum force. The cooling may be in the form of a cooled fluid, such as below ambient temperature air or ambient temperature air having a temperature in the range of about 10 degrees C to about 25 degrees C, for example. An air knife may be an appropriate tool to provide the cooling. The cooling may be applied to the web 1200 while the web1 1200 is under a vacuum force, a reduced vacuum force, or no vacuum force. The heating step may be performed prior to the cooling step or the cooling step may be performed prior to the heating step. The cooling may be applied to the web 1200 while the web 1200 is under a vacuum force, a reduced vacuum force, or no vacuum force. The difference in temperature of the heating compared to the cooling being applied to the web 1200 may be in the range of about 5 degrees C to about 10 degrees C, for example. A range of the temperature of the heating may be in the range of about 30 degrees C to about 130 degrees C, for example. A range of the temperature of the cooling may be in the range of about 10 degrees C to about 25 degrees C, for example. In some instances, only heating or only cooling may be used.

Heating and/or cooling the web 1200 may cause the continuous fibers to reorient thereby creating loft, softness, and structural integrity in the web. After the heating and/or cooling steps, the web 1200 may pass through a reduced or no vacuum zone 1312 prior to being conveyed into the through-fluid bonding oven 1144. The moving porous member 1134 and the web 1200 may be heated in the reduced or no vacuum zone 1312, by a hot fluid or otherwise to preheat the web 1200 before entering the through-fluid bonding oven 1144. The heating and/or cooling and reduced or no vacuum steps may be repeated any suitable number of times prior to conveying the web 1200 into a through-fluid bonding oven or other oven to achieve the desired results of loft, softness, and structural integrity. The continuous fiber nonwoven web 1200 may then be conveyed through and out of the through-fluid bonding oven 1144 to another process, such as winding 1332 or further bonding in another through-fluid bonding oven, for example.

Intermittently varying the vacuum forces applied to a web as discussed herein with respect to Figs. 14-16, also may encompass the vacuum forces being always "on", but may be gradually reduced or sequentially decreased as the web 1200 travels from the first zone 1206 towards the hot fluid supply 1217 and the hot fluid vacuum 1219. For example, a first zone may have the greatest vacuum force, the second zone may have a lesser vacuum force than the first zone, a third zone may have a lesser vacuum force than the second zone, a fourth zone may have a lesser vacuum force than the third zone, and so on depending on how many zones are present. The process described in this paragraph may achieve nonwoven webs with better loft and softness, without fuzzing and giving up strength.

Example 1: Method of Making

Round bicomponent molten polymers comprising 70% by weight of polyethylene and 30% by weight of polyester terephthalate, in a side-by-side configuration, were extruded vertically downward from a plurality of orifices of a spinneret and at a mass throughput of about 0.4 grams per orifice per minute. The resulting continuous fiber strands were quenched symmetrically by transverse flows of air cooled to about 15 degrees C, drawn by a high-velocity (> 25 m/s) air stream down to a fiber diameter of about 17 $\mu$m and directed by the air stream onto a moving porous member to create an intermediate continuous fiber nonwoven web on the moving porous member. The moving porous member was located about 2 meters below the spinneret. The intermediate continuous fiber nonwoven web had a basis weight of about 25 gsm. The moving porous member was 156 centimeters long and had ten zones in the machine direction. Table 1 below shows the machine direction length (cm) of the various zones and air flow (m/s) in each zone. For clarity, zone 1 is upstream of zone 2, zone 2 is upstream of zone 3 etc. Also for clarity, air speed is the speed of air flowing down through the moving porous member without the intermediate nonwoven web on the moving porous member as described herein.

Table 1:

| Zone | Length (cm) | Vacuum (m/s) |
|------|-------------|--------------|
| 1 | 10 | 18 |
| 2 | 10 | 10 |
| 3 | 10 | 4 |
| 4 | 15 | 2 1/2 |
| 5 | 10 | 0 |
| 6 | 5 | 5 |
| 7 | 51 | 0 |
| 8 | 5 | 6 |
| 9 | 20 | 0 |
| 10 | 20 | 1 1/4 |

In zone 10, the intermediate continuous fiber nonwoven web was lightly bonded with air that was heated to about 115 degrees C using an air heater that was located about 5.5 cm above the moving porous member. The air heater had an air flow rate of about 0.7 m/s. The intermediate continuous fiber nonwoven web was then through-fluid bonded in a through-fluid bonding oven.

Example 2:

A process identical to that described above in Example 1 was used to create continuous fiber strands and deposit them onto a moving porous member to create an intermediate continuous fiber nonwoven web having a basis weight of about 25 gsm. The 156-centimeter long moving porous member, however, had only six zones in the machine direction, distinguished either by changes in air flow or presence of an air heater. Table 2 below shows the machine direction length (cm), air flow (m/s) and air heater presence of the various zones. For clarity, zone 1 is upstream of zone 2, zone 2 is upstream of zone 3, zones 3 is upstream of zone 4, etc. Also for clarity, air speed is the speed of air flowing down through the moving porous member without the intermediate nonwoven web on the moving porous member as described herein. Note that the first vacuum force of 15 m/s was sequentially decreased to 1.5 m/s across different zones along the moving porous member.

Table 2:

| Zone | Length (cm) | Vacuum (m/s) | Air Heater |
|------|-------------|--------------|------------|
| 1 | 10 | 15 | No |
| 2 | 10 | 9 | No |
| 3 | 10 | 6 | No |
| 4 | 10 | 2 | No |
| 5 | 76 | 1.5 | No |
| 6 | 40 | 1.5 | 112°C |

In zone 6, the intermediate continuous fiber nonwoven web was lightly bonded with air that was heated to about 112 degrees C using an air heater that was located about 6.5 cm above the moving porous member. The air heater had an air flow rate of about 1.5 m/s. The lightly bonded intermediate continuous fiber nonwoven web was then through-fluid bonded in a through-fluid bonding oven, as described herein. The process described above may achieve nonwoven webs with better loft and softness, without fuzzing or giving up strength.

Example 3:

A process identical to that described above in Example 1 was used to create continuous fiber strands and deposit them onto a moving porous member to create an intermediate continuous fiber nonwoven web having a basis weight of about 25 gsm. The 156-centimeter long moving porous member, however, had twelve zones in the machine direction, distinguished either by changes in air flow or presence of an air heater. Table 3 below shows the machine direction length (cm), air flow (m/s) and air heater presence of the various zones. For clarity, zone 1 is upstream of zone 2, zone 2 is upstream of zone 3, zone 3 is upstream of zone 4, etc. Also for clarity, air speed is the speed of air flowing down through the moving porous member without the intermediate nonwoven web on the moving porous member as described herein. Note that the intermediate continuous fiber nonwoven web was exposed to several thermal cycles across different zones along the moving porous member.

Table 3:

| Zone | Length (cm) | Vacuum (m/s) | Air Heater |
| --- | --- | --- | --- |
| 1 | 10 | 15 | No |
| 2 | 10 | 9 | No |
| 3 | 10 | 6 | No |
| 4 | 10 | 2 | No |
| 5 | 20 | 1.5 | No |
| 6 | 10 | 1.5 | 80°C |
| 7 | 16 | 1.5 | No |
| 8 | 10 | 1.5 | 80°C |
| 9 | 20 | 1.5 | No |
| 10 | 10 | 1.5 | 124°C |
| 11 | 20 | 1.5 | No |
| 12 | 10 | 1.5 | 124°C |

In zones 6, 8 10 and 12, the intermediate continuous fiber nonwoven web was lightly bonded with air that was heated to either about 80°C (zones 6 and 8) or about 124°C (zones 10 and 12) using air heaters located about 6.5 cm above the moving porous member. The air heaters had an air flow rate of about 1.5 m/s. The lightly bonded intermediate continuous fiber nonwoven web was then through-fluid bonded in a through-fluid bonding oven, as described herein. This thermal cycling (or intermittently providing energy, heat, or hot air) in various zones may use a fluid or air having a temperature in the range of about 30 degrees C to about 130 degrees C, about 50 degrees C to about 130 degrees C, or about 70 degrees C to about 130 degrees C, for example. Other temperatures may also be suitable depending on the desired resulting web. The thermal cycling may occur during the intermittently varying the vacuum step or during the vacuum being sequentially decreased. Residence time during each thermal cycle (e.g., in a certain zone) may be in the range of about 0.1 seconds to about 2 seconds, about 0.1 seconds to about 1.5 seconds, or about 0.1 seconds to about 1 second, for example. The process described above may achieve nonwoven webs with better loft and softness, without fuzzing or giving up strength.

Through-Fluid Bonded Continuous Fiber Nonwoven Characteristics

The produced through-fluid bonded continuous fiber nonwoven webs may have certain characteristics that relate to loft, softness, and low fuzz. The continuous fiber nonwoven webs disclosed herein may form portions of absorbent articles, such as diapers, pants, sanitary napkins, and/or liners, for example. The continuous fiber nonwoven webs disclosed here may also form portions of, or all of, wipes, other consumer products, or other products.

*Martindale Average Abrasion Resistance (Fuzz Level)*

The through-fluid continuous fiber nonwoven webs of the present disclosure may have a Martindale Average Abrasion Resistance Grade in the range of about 1.0 to about 3.0, about 1.0 to about 2.9, about 1.0 to about 2.8, about 1.0 to about 2.7, about 1.0 to about 2.5, about 1.0 to about 2.5, about 1.0 to about 2.4, about 1.0 to about 2.3, about 1.0 to about 2.2, about 1.0 to about 2.1, about 1.0 to about 2.4, about 1.0 to about 2.3, about 1.0 to about 2.2, about 1.0 to about 2.1, about 1.0 to about 2.0, about 1.0 to about 1.5, about 1.0 to about 1.3, about 1.0 to about 1.2, about 1.3, about 1.2, or about 1.0, according to the Martindale Abrasion Resistance Grade Test herein, specifically reciting all 0.1increments within the specified ranges and all ranges formed therein or thereby.

*DMA Compression Resiliency*

The through-fluid bonded continuous fiber nonwoven webs of the present disclosure may have a DMA Compression Resiliency in the range of about 25% to about 90%, about 25% to about 70%, about 30% to about 70%, about 25% to about 50%, about 25% to about 40%, about 30% to about 40%, or about 30% to about 50%, according to the DMA Compression Resiliency Test herein, specifically reciting all 0.1% increments within the specified ranges and all ranges formed therein or thereby.

*Thickness*

The through-fluid continuous fiber nonwoven webs of the present disclosure may have a thickness in the range of about 0.5mm to about 5.0mm, about 0.5mm to about 3mm, about 0.5mm to about 2.5mm, about 0.5mm to about 2mm, about 0.75mm to about 3.0mm, about 0.8mm to about 2.0mm, about 0.9mm to about 1.5mm, according to the Thickness Test herein specifically reciting all $0.1\mu m$ increments within the specified ranges and all ranges formed therein or thereby.

*Basis Weight*

The through-fluid bonded continuous fiber nonwoven webs of the present disclosure may have a Basis Weight in the range of about 10 gsm to about 100 gsm, about 14 gsm to about 80 gsm, about 15 gsm to about 40 gsm, about 15 gsm to about 30 gsm, about 20 to about 30 gsm, or about 20 to about 25 gsm, according to the Basis Weight Test herein, specifically reciting all 0.1 gsm increments within the specified ranges and all ranges formed therein or thereby.

*Specific Nonwoven Volume*

Specific Nonwoven Volume is defined herein as the Thickness, measured by the Thickness Test, divided by the Basis Weight, measured by the Basis Weight Test. The through-fluid bonded continuous fiber nonwoven webs of the present disclosure may have a Specific Nonwoven Volume in the range of about 20 $cm^3$/g to about 100 $cm^3$/g, about 25 $cm^3$/g to about 100 $cm^3$/g, about 25 $cm^3$/g to about 80 $cm^3$/g, about 25 $cm^3$/g to about 60 $cm^3$/g, about 30 $cm^3$/g to about 100 $cm^3$/g, about 30 $cm^3$/g to about 80 $cm^3$/g, or about 25 $cm^3$/g to about 55 $cm^3$/g, specifically reciting all 0.1 $cm^3$/g increments within the specified ranges and all ranges formed therein or thereby.

Example 4:

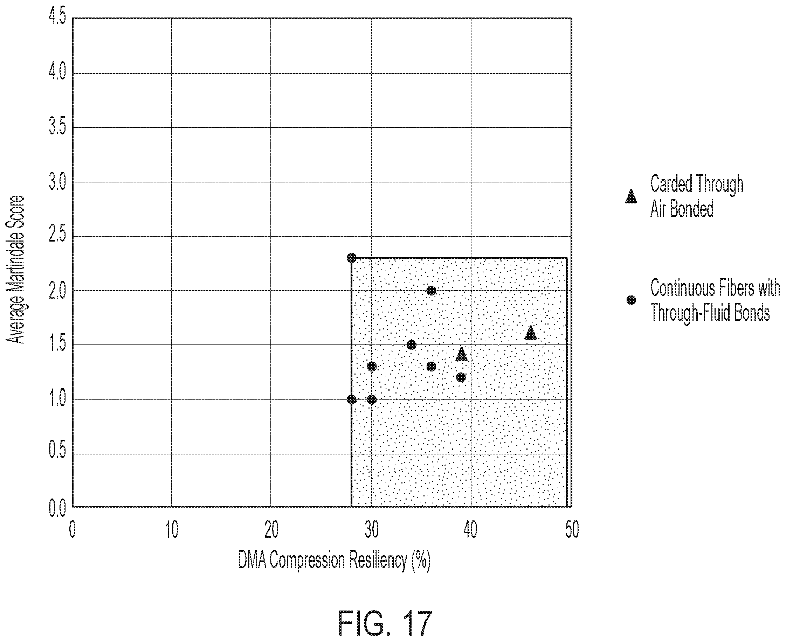

Soft and lofty nonwoven webs with limited fuzz and good structural integrity are desired. Fuzz is measured by the Martindale Average Abrasion Resistance Grade and loftiness and structural integrity are measured by the DMA Compression Resiliency Test. Fig. 17 is a graph of the Martindale Average Abrasion Resistance Grade (y-axis) vs. DMA Compression Resiliency % (x-axis). Samples 1-8 of the present disclosure were tested against two related art samples having carded fibers. One goal of the through-fluid bonded continuous fiber nonwoven webs of the present disclosure is to perform parity to carded webs in softness, loft, fuzzing, and structural integrity. The samples had the following characteristics as detailed in Table 4.

Table 4:

| Sample | Fiber Type | Final Bond Type | Basis Weight (gsm) | Thickness (mm) | Specific Nonwoven Volume (cm$^3$/g) | DMA Compression Resiliency % | Martindale Average Abrasion Resistance Grade |
|---|---|---|---|---|---|---|---|
| Present Disclosure 1 | Continuous PE/PET/ side by side | Through-air | 22 | 0.99 | 45 | 36 | 1.3 |
| Present Disclosure 2 | Continuous PE/PET side by side | Through-air | 25 | 1.33 | 53 | 30 | 1.3 |
| Present Disclosure 3 | Continuous PE/PET side by side | Through-air | 22 | 1.01 | 45 | 39 | 1.2 |
| Present Disclosure 4 | Continuous PE/PET side by side | Through-air | 25 | 1.34 | 54 | 30 | 1.0 |
| Present Disclosure 5 | Continuous PE/PET side by side | Through-air | 25 | 1.12 | 46 | 36 | 2.0 |
| Present Disclosure 6 | Continuous PE/PP side by side | Through-air | 27 | 0.98 | 36 | 28 | 1.0 |
| Present Disclosure 7 | Continuous PE/PET side by side | Through-air | 27 | 0.76 | 28 | 28 | 2.3 |
| Present Disclosure 8 | Continuous PE/PET sheath/ eccentric core | Through-air | 26 | 0.64 | 25 | 34 | 1.5 |
| Related Art 1 | Carded | Through-air | 19 | 0.38 | 20 | 39 | 1.4 |
| Related Art 2 | Carded | Through-air | 19 | 0.48 | 25 | 46 | 1.6 |

As can be seen from Table 4, samples 1-8 of the present disclosure achieve the benefits of a carded through-fluid bonded material, while being comprised of continuous fibers. As mentioned, continuous fiber nonwoven webs are much cheaper to manufacture than carded fiber nonwoven webs.

Test Methods

*Sample Conditioning*

Unless specifically noted below, all samples are conditioned at 23 ± 2° C and at 50 ± 2% relative humidity for 24 hours before testing.

*Thickness Test*

Thickness of a nonwoven web is measured using a ProGage Thickness Tester (Thwing-Albert Instrument Company, West Berlin, N.J.) with a pressure foot having a diameter of 2.221 inches (56.4mm) at a pressure of 0.5 kPa.

Five (5) samples are prepared by cutting of a usable sample such that each cut sample is at least 2.5 inches per side, avoiding creases, folds, and obvious defects. An individual specimen is placed on the anvil with the specimen centered underneath the pressure foot. The pressure foot is lowered at 0.03 inches/sec to an applied pressure of 0.5 kPa. The reading is taken after 3 seconds dwell time, and the pressure foot is raised. The measure is repeated in like fashion for the remaining 4 specimens. The thickness is calculated as the average caliper of the five specimens and is reported in mm to the nearest 0.01 mm.

*Specific Nonwoven Volume*

The Specific Nonwoven Volume is calculated from the thickness measurement and the Basis Weight measurement as:

$$\text{Specific Nonwoven Volume} = \frac{\text{Thickness (cm)}}{\text{Basis Weight (g/cm}^2)}$$

And reported to the nearest $cm^3$/g.

*DMA Compression Resiliency Test*

To measure the DMA Compression Resiliency of the through-fluid bonded continuous fiber nonwoven webs described herein, unconfined compression tests are performed on a TA Instruments Q800 DMA Dynamic Mechanical Analyzer (DMA) from TA Instruments - Waters LLC, New Castle, DE. The instrument is operated and calibrated as per the Operator Manual (DMA Dynamic Mechanical Analyzer Q Series Getting Started Guide Rev H, 2007) with the exceptions as listed below:

Samples are tested at ambient conditions, 22 $\pm$ 1°C and 43 $\pm$ 3% relative humidity. Three specimens are tested for each sample. Each specimen is cut with a hammer-driven (arch) punch having a diameter of 40 mm.

The 40 mm diameter compression plate fixtures (Compression plates provided in Parallel Plate Compression Clamp kit, Part Number 984018901, TA Instruments) are installed per the Operator Manual so that the flat surfaces are aligned and parallel. The instrument is calibrated according to the Operator Manual in three steps. In Clamp Mass Calibration, the mass of the clamp is tared out; in Clamp Zero Calibration, the instrument brings the two plates into contact and assigns to that position a gap of 0 mm; and in Clamp Compliance Calibration, the compliance of the compression assembly is measured in $\mu$m/N.

For the test, the data acquisition rate is set to 2 Hz (0.5 sec/data point). The compression plates are separated manually to a gap about 2-3 mm greater than the unrestrained thickness of the test specimen, and the as-prepared specimen is then inserted and centered between the plates. A pre-load force of 0.1256 N is applied, and a starting specimen height is measured by the instrument.

The test is then started, during which the specimen is subjected to a preprogramed force profile. The profile is such that the sample is compressed in Controlled-Force Mode by a sequence of six forces, each force being applied for 10 seconds (0.17 min). The sequence of forces and corresponding pressures are listed in the table below:

| Step | Force (N) | Pressure (psi) |
|------|-----------|----------------|
| 1 | 0.87 | 0.10 |
| 2 | 2.61 | 0.30 |
| 3 | 4.35 | 0.50 |
| 4 | 6.09 | 0.70 |
| 5 | 8.70 | 1.00 |
| 6 | 0.87 | 0.10 |

During the test, the specimen height is recorded by the instrument at each pressure in the table. The following parameters are calculated from the height at each pressure and reported:

DMA Compression Resiliency is calculated as the percent change in specimen height from 0.10 psi to 0.30 psi, i.e.,

$$100 * (h_1 - h_2) / h_1,$$

where

h$_1$ = average height of specimen during its 10 seconds at 0.10 psi during Step 1, and
h2 = average height of specimen during its 10 seconds at 0.30 psi during Step 2.

DMA Compression Recovery is calculated as the percent of specimen height measured during Step 1 attained during Step 6, i.e.,

$$100 * h_6 / h_1,$$

where

h$_6$ = average height of specimen during its 10 seconds at 0.10 psi during Step 6, and
h$_1$ = average height of specimen during its 10 seconds at 0.10 psi during Step 1.

*Basis Weight Test*

Basis weight of the through-fluid bonded continuous fiber nonwoven webs may be determined by several available techniques, but a simple representative technique involves taking an absorbent article or other consumer product, removing any elastic which may be present and stretching the absorbent article or other consumer product to its full length. A punch die having an area of 45.6 cm$^2$ is then used to cut a piece of the through-fluid bonded continuous fiber nonwoven webs (e.g., topsheet, outer cover) from the approximate center of the absorbent article or other consumer product in a location which avoids to the greatest extent possible any adhesive which may be used to fasten the through-fluid bonded continuous fiber nonwoven web to any other layers which may be present and removing the through-fluid bonded continuous fiber nonwoven web from other layers (using cryogenic spray, such as Cyto-Freeze, Control Company, Houston, Texas, if needed). The sample is then weighed and dividing by the area of the punch die yields the basis weight of the through-fluid bonded continuous fiber nonwoven web. Results are reported as a mean of 5 samples to the nearest g/m$^2$, which may be abbreviated as "gsm" herein.

*Martindale Average Abrasion Resistance Grade Test*

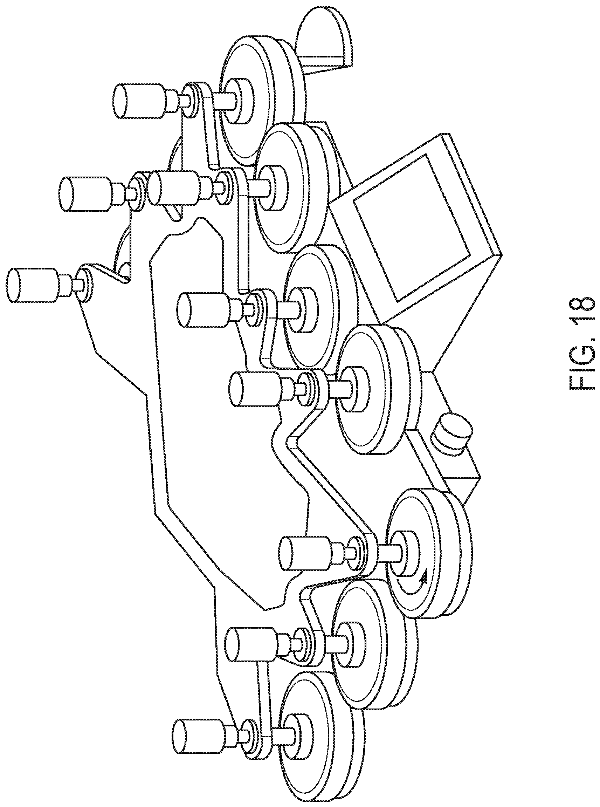

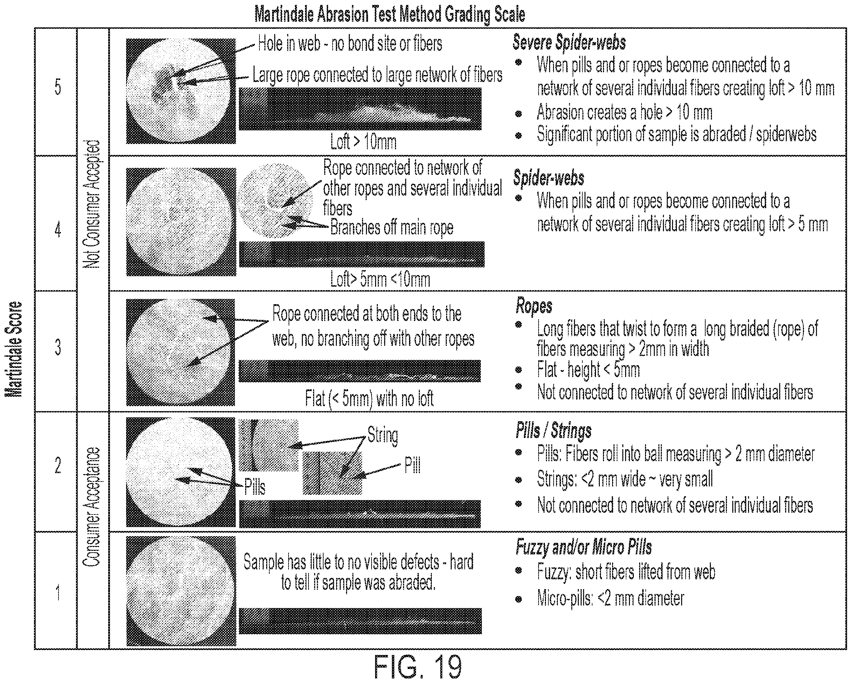

Fig. 18 is a perspective view of equipment for the Martindale Average Abrasion Resistance Grade Test. Fig. 19 is a grade scale for fuzz assessment in the Martindale Average Abrasion Resistance Grade Test herein.
Martindale Average Abrasion Resistance Grade of a nonwoven is measured using a Martindale Abrasion Tester (Model # 864, Nu Martindale Abrasion and Pilling Tester, James H. Heal & Co. Ltd. England). The Martindale Abrasion Tester is operated per instructions in Operator's Guide Publication 290-864$A from James H. Heal with the following modifications as below:

1) The test is conducted dry.
2) Nonwoven samples are conditioned for 24 hours at 23 $\pm$ 2° C and at 50 $\pm$ 2% relative humidity.
3) From each nonwoven sample, cut nine circular samples 6.375 inches in diameter. Cut a piece of Standard Felt (22 oz/yd$^2$ basis weight and 0.12 inches thick, obtained from James Heal) into a circle of 140 mm in diameter.
4) Secure each sample on each testing abrading table position of the Martindale by first placing the cut felt, then the cut nonwoven sample. Then secure the clamping ring, so no wrinkles are visible on the nonwoven sample.
5) Assemble the abradant holder. The abradant is a 38 mm diameter FDA compliant silicone rubber 1/32 inch thick (obtained from McMaster Carr, Item 86045K21-50A). Place the required weight in the abradant holder to apply 9 kPa pressure to the sample. Place the assembled abradant holder in the Model #864 such that the abradant contacts the NW sample as directed in the Operator's Guide.
6) Operate the Martindale abrasion under conditions below:

Mode: Abrasion Test
Speed: 47.5 cycles per minute; and
Cycles: 3 samples at 20 cycles, 3 samples at 40 cycles, and 3 samples at 80 cycles. 7) After the test stops, place the abraded nonwoven on a smooth, matte, black surface and grade its fuzz level using the scale provided in Fig. 19. Each sample is evaluated by observing both from the top, to determine dimension and number of defects, and from the side, to determine the height of loft of the defects. A number from 1 to 5 is assigned based on best match with the grading scale. The Martindale Average Abrasion Resistance Grade is then calculated as the average rating of all nine samples (3 samples each at 20, 40, and 80 cycles) and reported to nearest tenth.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present disclosure have been illustrated and described, it would be obvious to those skilled in the art that various other changes and modifications may be made without departing from the scope of the present disclosure. It is therefore intended to cover in the appended claims all such changes and modifications that are within the scope of this disclosure.

**Claims**

1. An absorbent article (10) comprising:

   a topsheet (26);
   a backsheet (28);
   an absorbent core (30) positioned at least partially intermediate the topsheet (26) and the backsheet (28); and
   a through-fluid bonded nonwoven web, the nonwoven web comprising a plurality of bicomponent continuous fibers, wherein the bicomponent continuous fibers comprise a first polymer and a second polymer, wherein the first polymer has a first melting temperature, wherein the second polymer has a second melting temperature, and wherein the first melting temperature is at least 10 degrees C different than the second melting temperature, but less than 180 degrees C;
   wherein the nonwoven web has:

   a Martindale Average Abrasion Resistance Grade in the range of 1.0 to 2.5, according to the Martindale Abrasion Resistance Grade Test as described herein; and
   a Dynamic Mechanical Analyser, DMA, Compression Resiliency in the range of 25% to 90%, preferably 25% to 70%, and more preferably 25% to 50%, according to the Dynamic Mechanical Analyser, DMA, Compression Resiliency Test as described herein.

2. The absorbent article of Claim 1, wherein the nonwoven web has:

   a Thickness in the range of 0.5 mm to 3.0 mm, preferably 0.8 mm to 2.0 mm, according to the Thickness Test; and
   a Basis Weight in the range of 10 gsm to 100 gsm, preferably 14 gsm to 80 gsm, and more preferably 15 gsm to 40 gsm, according to the Basis Weight Test.

3. The absorbent article of Claim 1 or 2, wherein the nonwoven web has:

   a Specific Nonwoven Volume in the range of 25 cm$^3$/g to 100 cm$^3$/g,
   preferably, 25 cm$^3$/g to 80 cm$^3$/g, wherein Specific Nonwoven Volume is calculated from the Thickness measurement and the Basis Weight measurement as:

$$\text{Specific Nonwoven Volume} = \frac{\text{Thickness (cm)}}{\text{Basis Weight (g/cm}^2\text{)}}$$

   and reported to the nearest cm$^3$/g.

4. The absorbent article of any one of the preceding claims, wherein the bicomponent continuous fibers comprise polyethylene and polypropylene.

5. The absorbent article of any one of Claims 1-3, wherein the bicomponent continuous fibers comprise polyethylene and polyethylene terephthalate.

6. The absorbent article of any one of Claims 1-3, wherein the bicomponent continuous fibers comprise polyethylene and polylactic acid.

7. The absorbent article of any one of the preceding claims, wherein the absorbent article is a diaper, a pant, or an adult incontinence article.

8. The absorbent article of any one of the preceding claims, wherein the absorbent article is a sanitary napkin or a liner.

9. The absorbent article of any one of the preceding claims, wherein the topsheet comprises the nonwoven web.

10. The absorbent article of any one of Claims 1-8, wherein the absorbent article comprises an outer cover nonwoven material (40), and wherein the outer cover nonwoven material (40) comprises the nonwoven web.

11. The absorbent article of any one of the preceding claims, wherein the nonwoven web has a Martindale Average Abrasion Resistance Grade in the range of 1.0 to 2.3, according to the Martindale Abrasion Resistance Grade Test as described herein.

12. The absorbent article of any one of the preceding claims, wherein the nonwoven web has a DMA Compression Resiliency in the range of 25% to 50%, according to the Dynamic Mechanical Analyser, DMA, Compression Resiliency Test as described herein.

13. The absorbent article of any one of the preceding claims, wherein the nonwoven web has a Specific Nonwoven Volume in the range of 25 cm$^3$/g to 55 cm$^3$/g, preferably 45 cm$^3$/g to 55 cm$^3$/g.

14. The absorbent article of any one of the preceding claims, wherein the bicomponent continuous fibers have a decitex of 0.8 to 3.

**Patentansprüche**

1. Absorptionsartikel (10), umfassend:

   eine Oberschicht (26);
   eine Unterschicht (28);
   einen Absorptionskern (30), der zumindest teilweise zwischen der Oberschicht (26) und der Unterschicht (28) angeordnet ist; und
   eine fluidgebundene Vliesbahn, wobei die Vliesbahn eine Vielzahl von Bikomponenten-Endlosfasern umfasst, wobei die Bikomponenten-Endlosfasern ein erstes Polymer und ein zweites Polymer umfassen, wobei das erste Polymer eine erste Schmelztemperatur aufweist, wobei das zweite Polymer eine zweite Schmelztemperatur aufweist, und wobei die erste Schmelztemperatur um mindestens 10 °C, aber weniger als 180 °C, von der zweiten Schmelztemperatur abweicht;
   wobei die Vliesbahn aufweist:

   einen durchschnittlichen Martindale-Abnutzungsbeständigkeitsgrad im Bereich von 1,0 bis 2,5, gemäß der Martindale-Abnutzungsbeständigkeitsgradprüfung, wie hierin beschrieben; und
   eine DMA-Druckelastizität (DMA - Dynamic Mechanical Analyser) im Bereich von 25 % bis 90 %, vorzugsweise 25 % bis 70 % und stärker bevorzugt 25 % bis 50 %, gemäß der DMA-Druckelastizitätsprüfung (DMA - Dynamic Mechanical Analyser), wie hierin beschrieben.

2. Absorptionsartikel nach Anspruch 1, wobei die Vliesbahn aufweist:

   eine Dicke im Bereich von 0,5 mm bis 3,0 mm, vorzugsweise
   0,8 mm bis 2,0 mm, gemäß der Dickenprüfung; und
   ein Flächengewicht im Bereich von 10 g/m$^2$ bis 100 g/m$^2$, vorzugsweise 14 g/m$^2$ bis 80 g/m$^2$ und stärker bevorzugt 15 g/m$^2$ bis 40 g/m$^2$, gemäß der Flächengewichtsprüfung.

3. Absorptionsartikel nach Anspruch 1 oder 2, wobei die Vliesbahn aufweist:

ein spezifisches Vliesvolumen im Bereich von 25 cm$^3$/g bis 100 cm$^3$/g, vorzugsweise 25 cm$^3$/g bis 80 cm$^3$/g, wobei das spezifische Vliesvolumen aus der Dickenmessung und der Flächengewichtsmessung berechnet wird:

$$\text{Spezifisches Vliesvolumen} = \frac{\text{Dicke (cm)}}{\text{Flächengewicht (g/cm}^2)}$$

und angegeben wird als die nächsten cm$^3$/g.

4. Absorptionsartikel nach einem der vorstehenden Ansprüche, wobei die Bikomponenten-Endlosfasern Polyethylen und Polypropylen umfassen.

5. Absorptionsartikel nach einem der Ansprüche 1 bis 3, wobei die Bikomponenten-Endlosfasern Polyethylen und Polyethylenterephthalat umfassen.

6. Absorptionsartikel nach einem der Ansprüche 1 bis 3, wobei die Bikomponenten-Endlosfasern Polyethylen und Polymilchsäure umfassen.

7. Absorptionsartikel nach einem der vorstehenden Ansprüche, wobei der Absorptionsartikel eine Windel, ein Höschen oder ein Erwachseneninkontinenzartikel ist.

8. Absorptionsartikel nach einem der vorstehenden Ansprüche, wobei der Absorptionsartikel eine Damenbinde oder eine Einlage ist.

9. Absorptionsartikel nach einem der vorstehenden Ansprüche, wobei die Oberschicht die Vliesbahn umfasst.

10. Absorptionsartikel nach einem der Ansprüche 1 bis 8, wobei der Absorptionsartikel ein Außenmantelvliesmaterial (40) umfasst, und wobei das Außenmantelvliesmaterial (40) die Vliesbahn umfasst.

11. Absorptionsartikel nach einem der vorstehenden Ansprüche, wobei die Vliesbahn einen durchschnittlichen Martindale-Abnutzungsbeständigkeitsgrad im Bereich von 1,0 bis 2,3, gemäß der Martinindale-Abnutzungsbeständigkeitsgradprüfung, wie hierin beschrieben, aufweist.

12. Absorptionsartikel nach einem der vorstehenden Ansprüche, wobei die Vliesbahn eine DMA-Druckelastizität (DMA - Dynamic Mechanical Analyser) im Bereich von 25 % bis 50 %, gemäß der DMA-Druckelastizitätsprüfung (DMA - Dynamic Mechanical Analyser), wie hierin beschrieben, aufweist.

13. Absorptionsartikel nach einem der vorstehenden Ansprüche, wobei die Vliesbahn ein spezifisches Vliesvolumen im Bereich von 25 cm$^3$/g bis 55 cm$^3$/g, vorzugsweise 45 cm$^3$/g bis 55 cm$^3$/g, aufweist.

14. Absorptionsartikel nach einem der vorstehenden Ansprüche, wobei die Bikomponenten-Endlosfasern einen Decitex-Wert von 0,8 bis 3 aufweisen.

**Revendications**

1. Article absorbant (10) comprenant :

une feuille de dessus (26) ;
une feuille de fond (28) ;
une âme absorbante (30) positionnée au moins partiellement entre la feuille de dessus (26) et la feuille de fond (28) ; et
une nappe non tissée liée par circulation de fluide, la nappe non tissée comprenant une pluralité de fibres continues bicomposantes, dans lequel les fibres continues bicomposantes comprennent un premier polymère et un deuxième polymère, dans lequel le premier polymère a une première température de fusion, dans lequel le deuxième polymère a une deuxième température de fusion, et dans lequel la première température de fusion est différente d'au moins 10 degrés C par rapport à la deuxième température de fusion, mais inférieure à 180 degrés C ;
dans lequel la nappe non tissée a :

un grade de résistance moyenne à l'abrasion Martindale dans la plage de 1,0 à 2,5, selon le test de grade de résistance à l'abrasion Martindale tel que décrit ici ; et
une résilience à la compression par analyseur mécanique dynamique, DMA, dans la plage de 25 % à 90 %, de préférence 25 % à 70 %, et plus préférablement 25 % à 50 %, selon le test de résilience à la compression par analyseur mécanique dynamique, DMA tel que décrit ici.

2.  Article absorbant selon la revendication 1, dans lequel la nappe non tissée a :

une épaisseur dans la plage de 0,5 mm à 3,0 mm, de préférence
0,8 mm à 2,0 mm, selon le test d'épaisseur ; et
une masse surfacique dans la plage de 10 g/m$^2$ à 100 g/m$^2$, de préférence 14 g/m$^2$ à 80 g/m$^2$, et plus préférablement 15 g/m$^2$ à 40 g/m$^2$, selon le test de masse surfacique.

3.  Article absorbant selon la revendication 1 ou 2, dans lequel la nappe non tissée a :

un volume spécifique de non-tissé dans la plage de 25 cm$^3$/g à 100 cm$^3$/g,
de préférence, 25 cm$^3$/g à 80 cm$^3$/g dans lequel le volume spécifique de non-tissé est calculé à partir de la mesure d'épaisseur et de la mesure de masse surfacique en tant que :

Volume spécifique de non-tissé = Épaisseur (cm)/ Masse surfacique (g/cm$^2$)
et rapporté au cm$^3$/g le plus proche.

4.  Article absorbant selon l'une quelconque des revendications précédentes, dans lequel les fibres continues bicomposantes comprennent du polyéthylène et du polypropylène.

5.  Article absorbant selon l'une quelconque des revendications 1 à 3, dans lequel les fibres continues bicomposantes comprennent du polyéthylène et du téréphtalate de polyéthylène.

6.  Article absorbant selon l'une quelconque des revendications 1 à 3, dans lequel les fibres continues bicomposantes comprennent du polyéthylène et de l'acide polylactique.

7.  Article absorbant selon l'une quelconque des revendications précédentes, dans lequel l'article absorbant est une couche, une culotte, ou un article d'incontinence pour adulte.

8.  Article absorbant selon l'une quelconque des revendications précédentes, dans lequel l'article absorbant est une serviette hygiénique ou un protège-slip.

9.  Article absorbant selon l'une quelconque des revendications précédentes, dans lequel la feuille de dessus comprend la nappe non tissée.

10. Article absorbant selon l'une quelconque des revendications 1 à 8, dans lequel l'article absorbant comprend un matériau non tissé de couverture externe (40), et dans lequel le matériau non tissé de couverture externe (40) comprend la nappe non tissée.

11. Article absorbant selon l'une quelconque des revendications précédentes, dans lequel la nappe non tissée a un grade de résistance moyenne à l'abrasion Martindale dans la plage de 1,0 à 2,3, selon le test de grade de résistance à l'abrasion Martindale tel que décrit ici.

12. Article absorbant selon l'une quelconque des revendications précédentes, dans lequel la nappe non tissée a une résilience à la compression DMA dans la plage de 25 % à 50 %, selon le test de résilience à la compression par analyseur mécanique dynamique, DMA tel que décrit ici.

13. Article absorbant selon l'une quelconque des revendications précédentes, dans lequel la nappe non tissée a un volume spécifique de non-tissé dans la plage de 25 cm$^3$/g à 55 cm$^3$/g, de préférence 45 cm$^3$/g à 55 cm$^3$/g.

14. Article absorbant selon l'une quelconque des revendications précédentes, dans lequel les fibres continues bicomposantes ont un décitex de 0,8 à 3.

Fig. 1

Fig. 2

Fig. 3

Fig. 4

Fig. 5

Fig. 6

Fig. 7

Fig. 8

Fig. 9

Fig. 10

Fig. 11

Fig. 12

FIG. 12A

FIG. 13

EP 3 887 585 B1

FIG. 14

EP 3 887 585 B1

EP 3 887 585 B1

1222

1223    1225

| OFF | ON | OFF | ON | OFF | ON | OFF |
|-----|----|-----|----|-----|----|-----|

1224

FIG. 15

FIG. 16

FIG. 17

FIG. 18

## Martindale Abrasion Test Method Grading Scale

| Martindale Score | | | |
|---|---|---|---|
| | Not Consumer Accepted | 5 | Hole in web - no bond site or fibers — Large rope connected to large network of fibers — Loft > 10mm | **Severe Spider-webs** • When pills and or ropes become connected to a network of several individual fibers creating loft > 10 mm • Abrasion creates a hole > 10 mm • Significant portion of sample is abraded / spiderwebs |
| | | 4 | Rope connected to network of other ropes and several individual fibers — Branches off main rope — Loft> 5mm <10mm | **Spider-webs** • When pills and or ropes become connected to a network of several individual fibers creating loft > 5 mm |
| | | 3 | Rope connected at both ends to the web, no branching off with other ropes — Flat (< 5mm) with no loft | **Ropes** • Long fibers that twist to form a long braided (rope) of fibers measuring > 2mm in width • Flat - height < 5mm • Not connected to network of several individual fibers |
| | Consumer Acceptance | 2 | String — Pill — Pills | **Pills / Strings** • Pills: Fibers roll into ball measuring > 2 mm diameter • Strings: <2 mm wide ~ very small • Not connected to network of several individual fibers |
| | | 1 | Sample has little to no visible defects - hard to tell if sample was abraded. | **Fuzzy and/or Micro Pills** • Fuzzy: short fibers lifted from web • Micro-pills: <2 mm diameter |

FIG. 19

EP 3 887 585 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005001188 A
- US 20140005020
- US 9421137 B
- US 7901393 B
- US 9498389 B
- US 6645190 B
- US 8747379 B
- US 8372052 B
- US 8361048 B
- US 6761711 B
- US 6817994 B
- US 8007485 B
- US 7862550 B

- US 6969377 B
- US 7497851 B
- US 6849067 B
- US 6893426 B
- US 6953452 B
- US 6840928 B
- US 8579876 B
- US 7682349 B
- US 7156833 B
- US 7201744 B
- US 5628097 A, Benson
- US 20160136014 A, Arora
- US 10190244 B, Ashraf